Europäisches Patentamt

European Patent Office

Office européen des brevets

(19)

(11) EP 0 546 333 B1

(12) EUROPEAN PATENT SPECIFICATION

(45) Date of publication and mention
of the grant of the patent:
**04.02.1998 Bulletin 1998/06**

(51) Int Cl.6: **C12N 15/85**, C12P 21/00

(21) Application number: **92119203.5**

(22) Date of filing: **10.11.1992**

(54) **Cloned DNA having enhancer activity, recombinant vector comprising the same and process for producing gene product using the same**

DNS Klone mit 'Enhancer' Aktivität, rekombinanter Vektor der es enthält und Verfahren zur Herstellung von Genprodukten unter Verwendung desselben

Clone d'ADN ayant une activité de 'enhancer', vecteur recombinant le comprenant et procédé de production de produit genetique l'utilisant

(84) Designated Contracting States:
**BE DE FR GB**

(30) Priority: **03.12.1991 JP 348300/91**
**22.06.1992 JP 187477/92**

(43) Date of publication of application:
**16.06.1993 Bulletin 1993/24**

(73) Proprietor: **NICHIREI CORPORATION**
**Tokyo 104 (JP)**

(72) Inventors:
• **Oda, Kinichiro**
**Tokyo 145 (JP)**
• **Nakada, Susumu**
**Nagareyama-shi, Chiba 270-01 (JP)**
• **Nakajima, Takuma**
**Kawasaki-shi, Kanagawa 213 (JP)**
• **Nakamura, Takeshi**
**Shinnanyou-shi, Yamaguchi 746 (JP)**
• **Tsunoda, Shiho**
**Saitama 340 (JP)**

(74) Representative: **Tiedtke, Harro, Dipl.-Ing. et al**
**Patentanwaltsbüro**
**Tiedtke-Bühling-Kinne & Partner**
**Bavariaring 4**
**80336 München (DE)**

(56) References cited:
**WO-A-86/04920**

• **MOLECULAR AND CELLULAR BIOLOGY vol. 9, no. 4, 1989, WASHINGTON US, pages 1498-1506; Dean DC; Blakeley MS; Newby RF; Ghazal P; Hennighausen L; Bourgeois S 'Forskolin inducibility and tissue-specific expression of the fibronectin promoter.'**
• **JOURNAL OF BIOLOGICAL CHEMISTRY. (MICROFILMS), vol. 265, no. 6, 1990, BALTIMORE, MD US, pages 3001-3004; RANGANATHAN G; GETZ M J: 'COOPERATIVE STIMULATION OF SPECIFIC GENE TRANSCRIPTION BY EPIDERMAL GROWTH FACTOR AND TRANSFORMING GROWTH FACTOR TYPE BETA-1'**

**Description**

BACKGROUND OF THE INVENTION

I. Field of the Invention

This invention relates to a DNA having an enhancer activity for fibronectin (hereinafter also referred to as "FN") gene, which aids to efficiently express a structural gene in mammalian cells cultured in serum-free or low-serum medium.

II. Description of the Related Art

There are three types of processes commonly employed for the production of genetically engineered gene products, categorized by their using hosts; 1) the processes using bacteria such as Escherichia coli, 2) the processes using eumycetes such as yeast, 3) the processes using mammals or their cells. Whereas their large-scale productivity, the processes 1) and 2) have drawbacks in that (a) generally the gene products may not be secreted outside the cells, (b) the structure of the gene products originated from mammals may be changed, and (c) the glycosylation and/or phosphorylation of the gene products may be failed or incomplete; such modifications are intrinsic for the functions of the gene products originated from mammals. Therefore, for the production of the gene products originated from mammals, it is desired to employ mammalian cells.

The processes 3) are grouped into those employing mammalian individuals and those employing mammalian cells. With the processes utilizing mammalian individuals, the gene products may adversely affect the hosts (i.e., individuals which produce the desired gene products), and it is difficult to control the differences among the individuals. Thus, at present, the processes which utilize mammalian cells are usually employed.

For culturing mammalian cells for the purpose of producing a desired substance, the media containing a high concentration (5 - 20 vol%) of mammalian serum are employed. The sera added to such media are expensive. Further, their qualities vary depending on the lot, so that the user must check the quality of the serum to be used. Thus, the cost concerning the serum to be added to the culture medium accounts for the major part of the culturing cost. Further, in collecting a gene product expressed in the mammalian cells or a metabolite thereof (the gene product and its metabolite are hereinafter collectively referred to as "substance"), the serum components make it difficult to purify the desired substance so as to increase the cost required for the purification of the substance.

Thus, in the production of a substance utilizing cultured mammalian cells, to reduce the production cost, it is most effective to employ a serum-free medium or a medium containing serum at a very low concentration (i.e., 2.0 vol% or less) (hereinafter referred to as "low-serum medium)". However, there are many cells which do not adapt to the serum-free medium, and there are many genes which are not expressed with the decrease of the growth activities of the cells in a low-serum medium. Thus, problems exist in employing serum-free or low-serum media in practice.

Fibronectin (FN) which is a constituent of the extracellular matrix is very strongly expressed in normal type adhesive cells at quiescent period (the state at which the growth of the cells are inhibited by culturing the cells in a low-serum medium or by increasing the cell density so as to cause contact inhibition), and its expression is suppressed when the growth of the cells is initiated. The expression level of fibronectin depends on its activity of the regulatory gene (Hara et al., Gene 70, 97 (1988)). Therefore, by introducing a gene containing a cloned structural gene at a downstream region of the FN promoter, a cell line (transformant) may be established, which produce the gene product at a high level in a low-serum medium.

FN promoters have been cloned from several mammalian cells including rat and human, and their primary structures (nucleotide sequences) have been determined (Patel et al., The EMBO Journal, Vol. 6, No. 9, 2565, 1987).

In D.C. Dean et al., Mol. Cell. Biol., Vol. 9 (4), April 1989, pp. 1498 - 1506, the 5'-flanking regions of the human and rat fibronectin genes are disclosed. This reference reports on the tissue specific expression of the FN promoter due to a cAMP responsive element identified in the 5'-flanking region (-170) of the human FN gene and that this sequence can mediate cAMP induction in different human cell lines (see page 1503, bridging paragraph from the left column to the right column). Enhancer elements effective for the expression of the FN promoter are not described.

However, these analyses are not complete. For example, the enhancer which is necessary for the expression of FN promoter remains unknown. Further, there is no report about the study of FN promoter aiming at applying the FN promoter to the production of a substance, except for Oda et al., Saibo Kogaku Bessatsu 4, p3, 1988, in which the possibility of using the FN promoter for the production of a substance is suggested.

SUMMARY OF THE INVENTION

Accordingly, an object of the present invention is to provide a DNA having enhancer activity, by which a substance may be produced at a high level in mammalian cells in a serum-free or low-serum medium, as well as to provide uses thereof.

The present inventors intensively studied the cloning of the enhancer which functions under serum-free or low-serum condition. As a result, a DNA having enhancer activity for FN gene was obtained, and it was discovered that a structural gene may be efficiently expressed by using the DNA and the promoter, thereby completing the present invention.

That is, the present invention provides 1) a cloned DNA sequence derived from rat genomic DNA having an enhancer activity for the rat fibronectin gene; 2) a recombinant vector comprising operably linked promoter, a structural gene and said DNA of 1); and 3) a process for producing a gene product, comprising the step of culturing mammalian cells transformed with said recombinant vector of 2), and collecting the gene product produced by the expression of said structural gene.

By the present invention, a DNA having an enhancer activity for the rat FN gene was identified, separated and cloned, and a recombinant vector containing the same was provided. Since mammalian cells transformed with the recombinant vector of the present invention expresses a desired structural gene in serum-free or low-serum medium, the desired substance can be effectively produced by such transformed mammalian cells in serum-free or non-serum medium. Thus, by the present invention, it was made it possible to produce a substance using an inexpensive medium which does not contain serum and the purification step for removing the serum from the desired product is not necessary. Therefore, the desired product can be effectively produced at a low cost. Further, since transformed mammalian cells are used in the process of the present invention, gene products having modifications by sugar chains which are the same as or at least similar to those of the naturally occurring gene products may be produced.

## BRIEF DESCRIPTION OF THE DRAWINGS

Fig. 1 shows the results of Southern blotting of various fragments obtained by digesting $\lambda$-#908phage and $\lambda$-#916 phage clones and using the DNA fragment of -1068 nt to -531 nt of the rat FN 5' flanking region;

Fig. 2 shows gene maps of the plasmids prFNpp2.1 and prFNSn2.3 prepared by inserting the 5' flanking region containing rat FN promoter and enhancer into the multicloning site of pBluescript II KS+;

Fig. 3 shows the nucleotide sequence of a DNA containing rat FN promoter and enhancer region;

Fig. 4 shows gene maps of plasmid vectors pSV2CAT-BX and pSV2-XB which were used for analyzing the function of the rat FN promoter and enhancer regions;

Fig. 5 shows 26 kinds of FN promoters and enhancers in which no or various deletions or substitutions are made;

Fig. 6 shows a time table of CAT assay and temporary expression activity of FN enhancer and promoter;

Fig. 7 is a view which shows that the temporary expression of FN enhancer and promoter of XhoC cells is promoted by substitution of bases in the enhancer region;

Fig. 8 is a gene map of a transducing vector pF1900L;

Fig. 9 is a gene map of a transducing vector pF1900Lneo;

Fig. 10 is a gene map of a transducing vector pF$\Delta$b564L;

Fig. 11 is a gene map of a transducing vector pF$\Delta$b564Lneo;

Fig. 12A is a gene map of a transducing vector pF1900M;

Fig. 12B is a gene map of a human interferon gamma (HuIFN-$\gamma$) expression plasmid pF1900$\gamma$ constructed from a transducing vector pF1900M;

Fig. 13 shows the growth of I6, I7, I25 and I26 cell lines and production of HuIFN-$\gamma$ thereby;

Fig. 14 shows the change in the HuIFN-$\gamma$ concentration produced by I7 cell line wherein Fig. 14A shows the concentration change when the medium is not replaced and Fig. 14B shows the concentration change when the medium is replaced every other day;

Fig. 15 shows the results of Northern blot hybridization analysis wherein Fig. 15A shows the results of HuIFN-$\gamma$ mRNA expressed by I6, I7, I25 and I26 cells and Fig. 15B shows the results of $\beta$-actin mRNA (control experiment);

Fig. 16 shows the results of Southern blot hybridization analysis of pF1900$\gamma$ introduced into and retained by I6, I7, I25 and I26 cell lines; and

Fig. 17 shows the SDS-polyacrylamide gel electrophoresis image of HuIFN-$\gamma$ protein (separated by immunoprecipitation) produced by I6 and I7 cells.

## DETAILED DESCRIPTION OF THE PREFERRED EMBODIMENTS

The DNA according to the present invention, which has the enhancer activity for the FN gene is hereinafter also referred to as "enhancer DNA according to the present invention" or "FN enhancer".

The term "enhancer" herein means the nucleotide sequence in a DNA having the following features:

(1) The enhancer has an activity to increase the activity of the nearest promoter on the same DNA.

(2) The enhancer functions even if it is located considerably far (up to about 10,000 bases) from the gene acted by the enhancer.

(3) The enhancer does not need to be located at a prescribed position. That is, the enhancer does not need to be located upstream the 5' end of the gene, but may be located in the transcriptional region or downstream thereof.

(4) A type of enhancer sequence may prefer specific cells or may function only in the cells (tissue specificity).

By the above-mentioned features, enhancer can be distinguished from promoter.

The enhancer activity for the rat FN gene of the FN enhancer DNA according to the present invention herein means that the efficiency of the transcription of the rat FN promoter is increased, that is, the expression level of the structural gene under the control of the FN promoter is increased, if the enhancer DNA is ligated to an upstream region of the FN promoter. Further, the increase in the efficiency of transcription is prominent in quiescent cells (see above) and the transcription is rapidly suppressed with the initiation of the cell growth. This is also an important property of the enhancer DNA of the present invention.

The enhancer DNA of the present invention may be obtained, for example, as follows: First, a probe is prepared by the polymerase chain reaction (PCR) method based on the base sequence of the rat FN promoter (Patel et al., The EMBO Journal, $\underline{6(9)}$, 2565). Using the thus prepared probe, a DNA containing the rat FN promoter region and the enhancer region is cloned. The cloned DNA is digested with one or more restriction enzymes to obtain a DNA containing a part of the structural gene encoding FN and the upstream region of the FN gene up to about 2 kbp from the 5' end of the FN gene. The thus obtained DNA is then subcloned to a plasmid DNA, and a recombinant plasmid DNA having a chloramphenicol acetyl transferase (CAT) gene as a reporter gene at downstream of the 2 kbp DNA is constructed. Animal cells are transfected with this DNA and the CAT activities of the cells are determined. If CAT activity is observed, the enhancer which functions under serum-free or low-serum conditions is located within the 2 kbp DNA upstream the FN gene.

More particularly, the enhancer may be obtained, for example, based on the method of Patel et al. That is, the rat genome library (Tamkun, J.W. et al., Proc. Natl. Acad. Sci. USA, $\underline{81}$, 5140 (1984)) inserted in EMBL 3 type $\lambda$ phage vectors is screened using the cDNA as a probe. From the phage clones reacted with the probe, DNAs containing the 25 kb region downstream the 3' end of FN genomic gene are extracted. Using the 5' end region of the thus obtained FN genomic gene DNA as a probe, the similar cloning is repeated to obtain a series of FN genomic gene DNAs, a part thereof overlapping each other. The positions of the DNAs on the FN gene are determined by Southern blotting using the cDNA as a probe. Among these, the DNA which reacts with the probe of the 5' end of the FN cDNA is further analyzed by making restriction enzyme map, determining base sequence and/or by conducting the RNase protection test, so as to determine the transcription initiation site of the FN gene and a part of the promoter.

Rat fibroblast cell line 3Y1 (Kimura et al., Int. J. Cancer, $\underline{15,}$ 694 (1975)) gene library inserted in EMBL 3 type $\lambda$ phage vector is screened by using the part of the thus obtained promoter as a probe. DNAs are extracted from the phage clones which react with the probe, and restriction maps of the region of the inserted rat genes are prepared. The DNA fragments upstream the structural genes, e.g., 2.0 kb fragments may be cloned again to, for example, plasmid pBluescript II KS.

By determining the base sequence of the cloned DNA and comparing the base sequence with that of the cDNA, the position of the translation initiation codon on the gene can be determined.

Further, by the S1 mapping method (Berk, A. J. and Sharp, P.A., Cell, $\underline{2}$, 721 (1977)), the transcription initiation site of the gene may be determined.

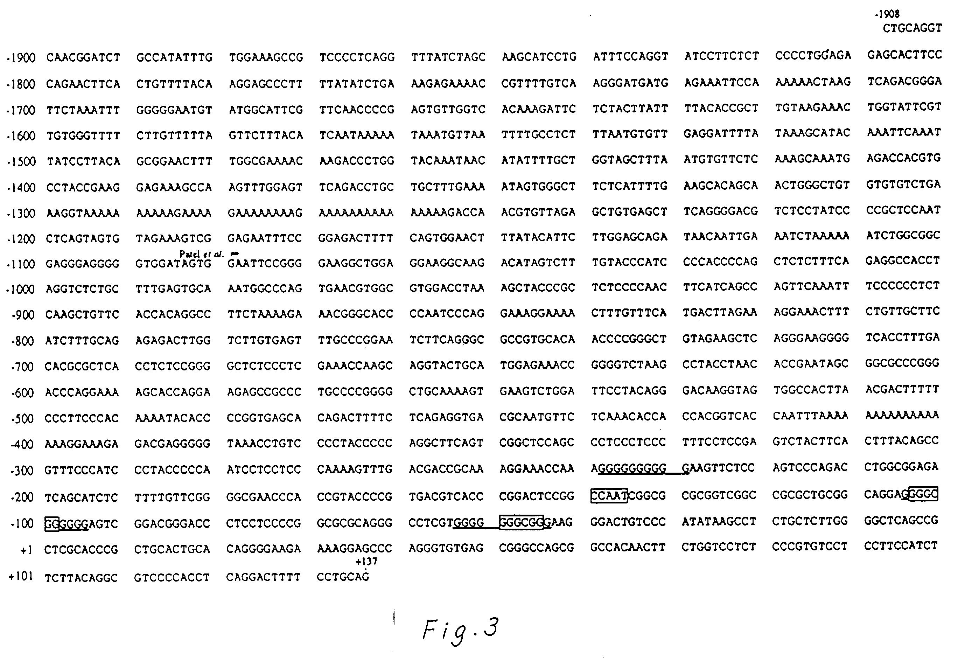

The enhancer DNA according to the present invention thus obtained is contained in the DNA fragment of -1908th to -1081th nucleotides in the DNA sequence shown in Fig. 3.

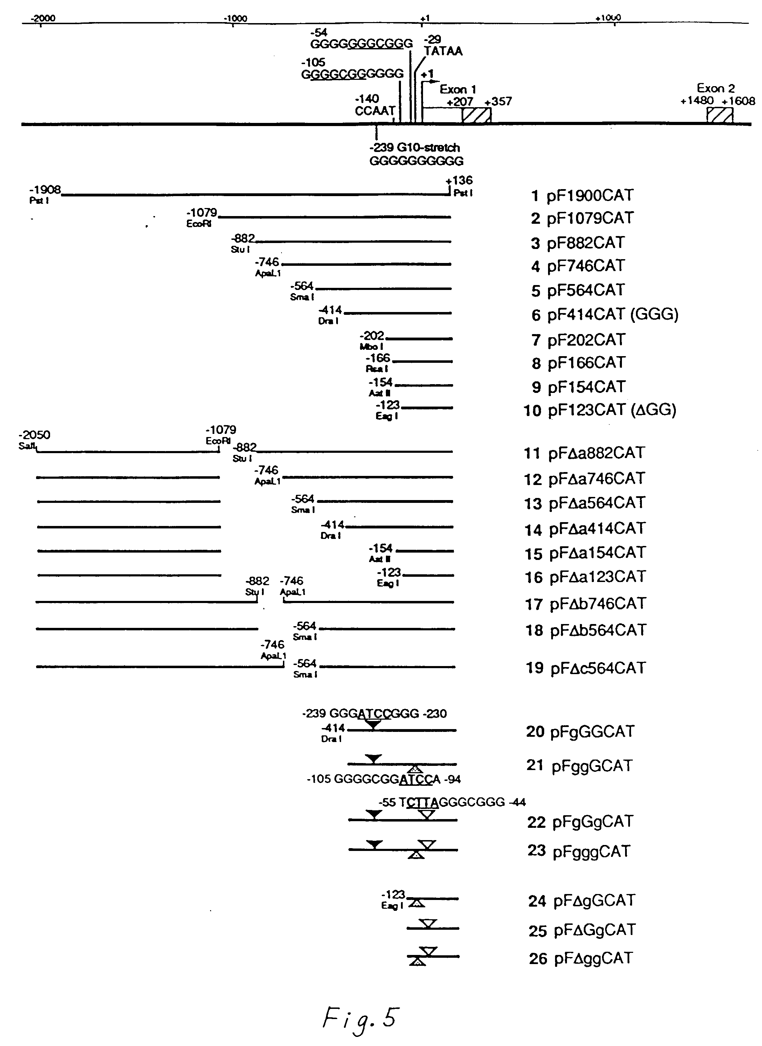

As the promoter, the DNAs containing "TATAA", "GGGCGG" and "CCAAT" shown in Fig. 5 of The EMBO Journal, $\underline{6}$, 2563-2572 (1987) may be employed.

The structural gene inserted in the recombinant vector may be derived from any species, and any structural gene which may be expressed in mammalian cells may be employed.

Examples of the structural gene include the gene encoding chloramphenicol acetyltransferase (CAT) originated from transposable element Tn9, the gene encoding human $\gamma$-interferon (IFN-$\gamma$) and human Nerve Growth Factor 2 (NGF-2) (see European Patent Publication No. 386,752A1).

The vector in which the enhancer DNA of the present invention, the promoter region and the structural gene are inserted may be any vector including vectors for mammalian cells such as pCD vector, cDM8 vector (Aruffo, A and Seed, B., Proc. Natl. Acad. Sci. USA, $\underline{84}$, 8573 (1987)), retrovirus vector (Cone, R. D. and Mulligan, R. C., Proc. Natl. Acad. Sci. USA, $\underline{81}$, 6349 (1984)); and other vectors such as pBR series and pUC series.

The enhancer DNA of the present invention, the promoter region and the structural gene may be inserted into the vector by the following procedure:

Firstly, a vector is digested with one or more appropriate restriction enzymes so that a foreign DNA can be inserted.

On the other hand, the FN enhancer DNA, the promoter region and the structural gene (assuming these are on the same DNA and successively arranged) are cut out from an appropriate source and, if necessary, the both ends of the cut out DNA fragment are processed. Thereafter, the vector and the DNA fragment containing the FN enhancer DNA, the promoter region and the structural gene are mixed and ligated by ligase.

The thus obtained recombinant vector is introduced into mammalian cells so as to transform the mammalian cells.

Preferred examples of the mammalian cell employed as a host include 3Y1 cell originated from rat fibroblast cell, 3T3 cell originated from mouse fibroblast cell, WI38 cell originated from human fibroblast cell, HUH7 cell originated from human hepatocarcinoma cell, and HmLu1 cell originated from hamster lung cancer cell. Among these, rat 3Y1 cell is best preferred.

The recombinant vector may be introduced into the mammalian cell by, for example, calcium phosphate method (Wigler, M. et al., Cell, 16, 777 (1979)), DEAE dextran method (Lopata, M.A. et al., Nucleic Acid Res., 12, 5707 (1984)), electroporation method (Ishizaki et al., Saibo Kogaku, Vol. 5, p.557, 1986) or microinjection method.

The mammalian cell transformant may be cultured by the conventional methods. Examples of the preferred media include MEM medium (Science, 122, 501 (1952)), DMEM medium (Virology, 8, 396 (1959)), RPMI 1640 medium (The Journal of the American Medical Association, 199, 519 (1967)) and 199 medium (Proceedings of the Society for the Biological Medicine, 73, 1 (1950)). The pH of the medium is preferably about 6 - 8. The culturing may usually be carried out at about 30 - 40°C for about 15 - 96 hours. If necessary, the culture medium may be aerated and/or stirred. The transformants may be cultured firstly in a medium containing serum and then transferred to a serum-free or a low-serum medium.

In the initial culture, a serum-free medium or a medium containing 0.1 - 20 vol% of bovine serum may be employed. Usually, a serum concentration of 10 vol% is sufficient (or 5 vol% is sufficient for some cells). After growing the cells to an appropriate number in these conditions, the cells are then transferred to a serum-free or low-serum (about 0.1 - 2 vol%) medium. By culturing the cells in the serum-free or the low-serum medium, the desired gene product may be obtained in a large amount. It is preferred to exchange the medium with a fresh medium, for example, every 3 days. The serum may be, for example, calf serum and fetal calf serum and the like, and the fetal calf serum is best preferred.

By the method described above, an arbitrary desired structural gene may be efficiently expressed under serum-free or low-serum condition, using the enhancer DNA of the present invention and the promoter.

The recombinant vector pF1900L containing the enhancer DNA of the present invention and the promoter, which was obtained in Example 1(3) described below, was deposited with Fermentation Research Institute of Japan on September 9, 1991 under the Budapest Treaty under the accession number FERM BP-3552.

The invention will now be described by way of examples thereof. It should be noted that the examples are presented for the illustration purpose only and should not be interpreted in any restrictive way.

Example 1

(1) Isolation of Rat FN Promoter and Enhancer

A genomic DNA library was prepared from rat fibroblast cell line 3Y1 clone b 1-6 ((Kimura et al., Int. J. Cancer 15, 694 (1975)), hereinafter referred to as "3Y1" for short) using EMBL3 type λ phage vector (commercially available from Stratagene Inc. The phage vector is hereinafter referred to as "Stg"). A DNA fragment having the sequence of from -1067th nucleotide (the nucleotide number is hereinafter expressed such as "-1067 nt") to -529 nt in the 5' flanking region of FN, which sequence was determined by Patel (the transcription initiation site is numbered as +1 nt and the nucleotide immediately upstream the +1 nt is numbered as -1 nt) was prepared by the polymerase chain reaction (hereinafter referred to as "PCR") method using the 3Y1 genomic DNA as a template.

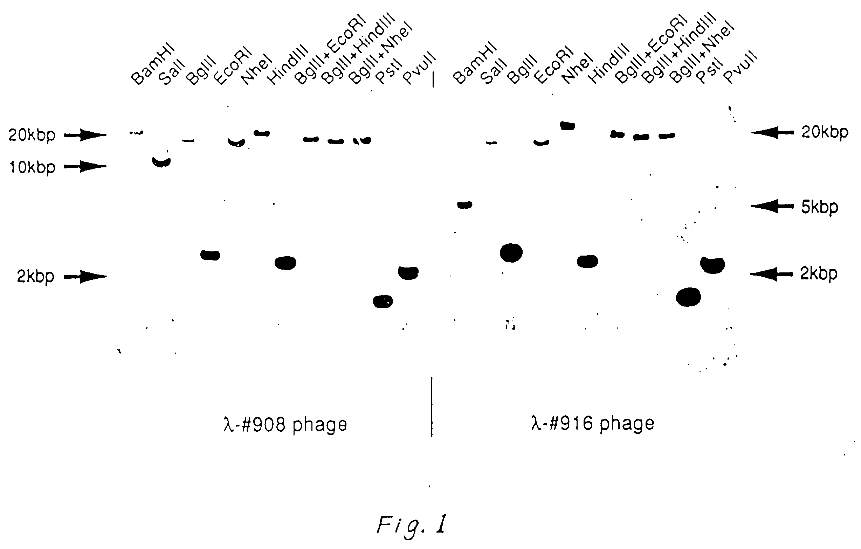

The 5'-end of the thus obtained DNA fragment was radio-labelled using ($\gamma$-$^{32}$P)ATP and T4 polynucleotide kinase to obtain a DNA probe. Using the thus prepared DNA probe, 1,500,000 clones of the above-mentioned genomic DNA library were screened to obtain two clones #908 and #916 which contain the FN promoter. Southern blotting analysis revealed that the clone #908 contained a Sal I fragment of about 10 kbp including the 5' flanking region of FN and the clone #916 contained a Sal I fragment of about 5.7 kbp including the 5' flanking region of FN (Fig. 1).

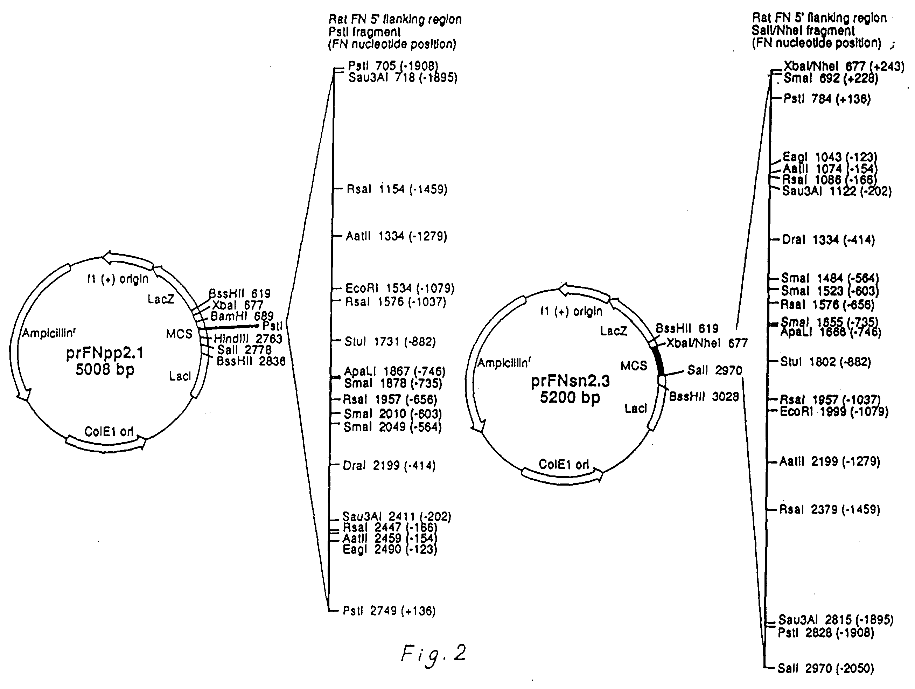

From the clone #908, a Pst I fragment of 2.1 kbp and a Sal I- Nhe I fragment of 2.3 kbp which contain the 5' flanking region of FN were cut out, and these fragments are subcloned to a plasmid pBluescript IIKS + (Stg) to obtain prFNpp2.1 and prFNsn2.3, respectively (Fig. 2). The base sequence of prFNpp2.1 was determined. As a result, it was found that prFNpp2.1 contained the nucleotide sequence of from -1908 nt to -1081 nt which has not been reported (Fig. 3. It should be noted that the nucleotide sequence from -1080 nt to +2460 nt has been reported by Patel et al (The EMBO Journal, 6(9), 2565 (1987)). The base sequence and the restriction map of the region downstream from -1080 nt were identical to those reported by Patel et al.

(2) Studies of Functional Regions of FN Promoter and Enhancer

The functional regions of FN promoter and enhancer were studied by the assay (CAT assay) of the temporary expression activity of chloramphenicol acetyltransferase (CAT assay). That is, various plasmids (pFCATs, see Fig. 5) containing FN 5' flanking region (containing promoter and enhancer) in which various deletions and substitutions of bases were introduced, which is inserted upstream the CAT gene, were prepared. 3Y1 clone b 1-6 was transfected with each of the pFCATs. Forty eight hours after the transfection, the CAT activities in the cell extracts were measured and the measured activities were compared each other.

(2)-1 Preparation of pFCATs

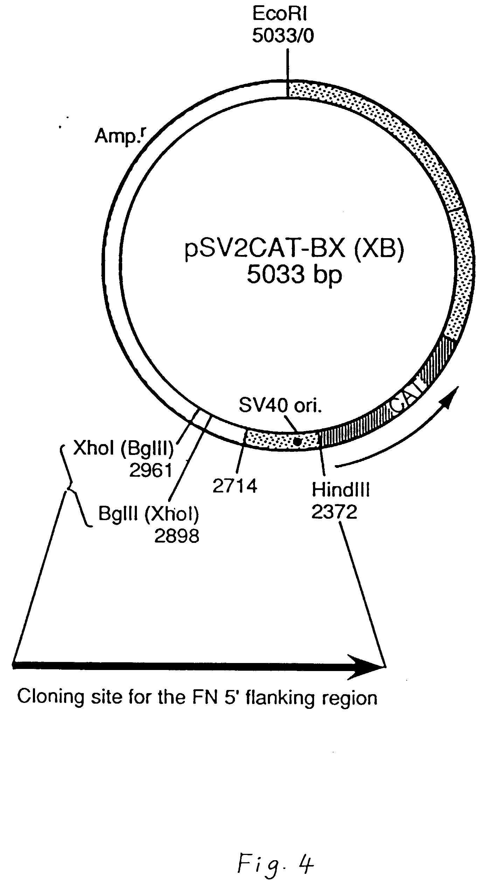

Firstly, in order to insert the FN promoter and enhancer at an upstream region of the CAT gene, plasmids pSV2CAT-BX and pSV2CAT-XB were prepared (Fig. 4). The plasmid pSV2CAT-BX is the same as the pSV2CAT reported by Gorman et al (Molecular and Cellular Biology, 2(9), 1044 (1982)) except that the Acc I restriction site and the Nde I restriction site are substituted by Bgl II site and Xho I site, respectively. The plasmid pSV2CAT-XB is the same as the pSV2CAT except that the Acc I restriction site and the Nde I restriction site are substituted by Xho I site and Bgl II site, respectively. FN 5' flanking region having various deletions and substitutions of bases were inserted upstream the CAT gene in the plasmids pSV2CAT-BX and pSV2CAT-XB to obtain 26 pFCATs (see Fig. 5. The prepared plasmids were numbered from 1 - 26 for convenience). The method for constructing each of these pFCAT plasmids will now be described.

pF1900CAT and pF202CAT (see Fig. 5; 1, 7)

Plasmid prFNpp2.1 was digested with Bam HI or Sau 3AI and Hind III to obtain a DNA fragment of 2.1 kbp (containing -1908 nt to +136 nt of FN 5, flanking region) and a DNA fragment of 351 bp (containing -202 nt to +136 nt of FN 5' flanking region). These DNA fragments were inserted between the Hind III site and Bgl II site of pSV2CAT-XB to obtain pF1900 CAT and pF202CAT, respectively.

pFC1079CAT (see Fig. 5; 2)

After digesting prFNsn2.3 with Eco RI, the resultant was blunt-ended with T4DNA polymerase and a Bam HI linker was attached. The resultant was digested with Bam HI and Pst I, and the obtained DNA fragment of 1.4 kbp (containing -1079 nt to +136 nt of FN 5, flanking region) was inserted between the Hind III site and Bgl II site of pSV2CAT-XB using a Hind III-Pst I adaptor (5'AGCTTGCA3') to obtain pF1079CAT.

pF882CAT, pF564CAT, pF414CAT and pF166CAT (see Fig. 5; 3, 5, 6, 8)

Plasmid prFNpp2.1 was digested with Stu I, Sma I, Dra I and Rsa I, and Bgl II linker was attached. The resulting DNA fragments sizing 1 kbp (containing -882 nt to +136 nt of FN 5' flanking region), 710 bp (containing -564 nt to +136 nt), 560 bp (containing -414 nt to +136 nt) and 310 bp (-166 nt to +136 nt), respectively, were inserted between the Hind III site and the Bgl II site of pSV2CAT-XB to obtain pF882CAT, pF564CAT, pF414CAT and pF166CAT, respectively.

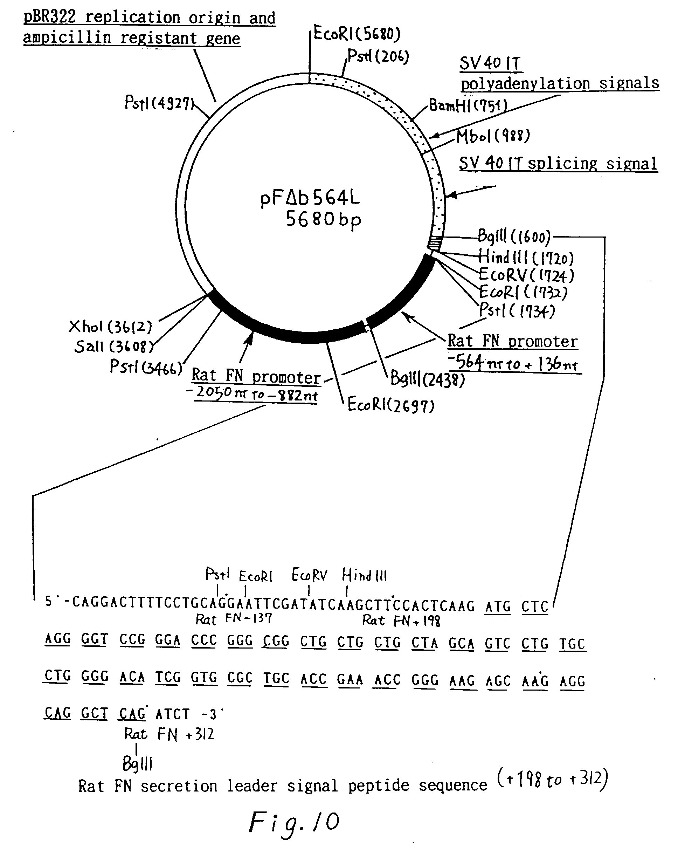

pF746CAT and pF123CAT (see Fig. 5; 4, 10)

Plasmid prFNpp2.1 was digested with Apa LI or Eag I and the resultants were blunt-ended by T4DNA polymerase, followed by addition of Bgl II linker (The Eag I site was repaired by the addition of the linker). The resultants were digested with Bgl II and Hind III to obtain a DNA fragment of 900 bp (containing -746 nt to +136 nt of the FN 5' flanking region) and a DNA fragment of 270 bp (-123 nt to +136 nt). Each of these DNA fragments was inserted between the Hind III site and the Bgl II site of pSV2CAT-XB to obtain pF746CAT and pF123CAT, respectively.

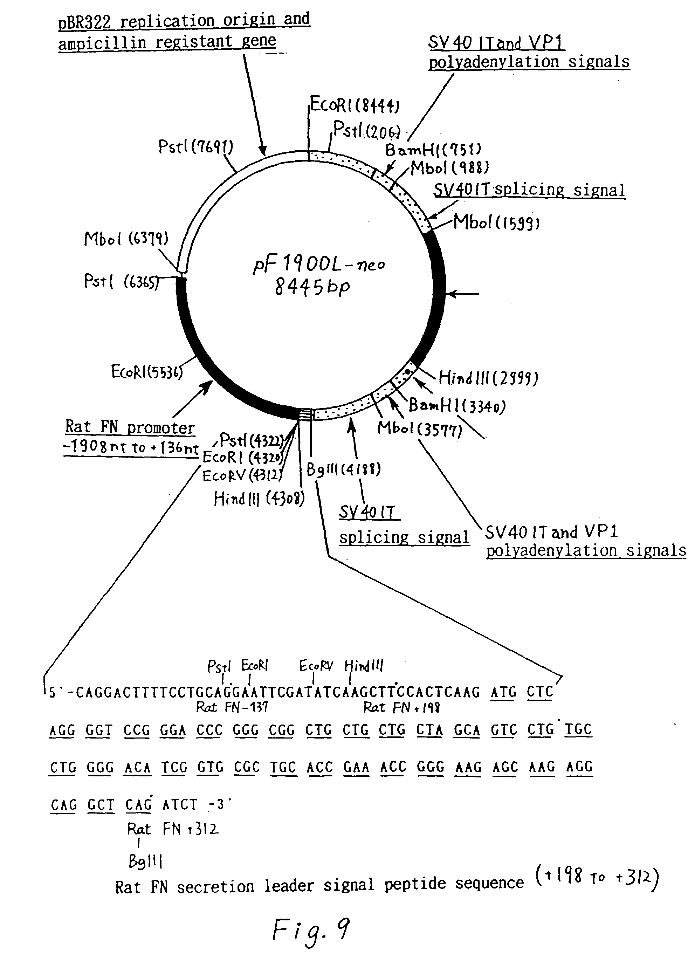

pF154CAT (see Fig. 5; 9)

Plasmid prFNpp2.1 was digested with Aat II and Hind III to obtain a DNA fragment of 300 bp (containing -154 nt to +136 nt of 5' flanking region of FN). The DNA fragment was inserted between the Hind III site and the Bgl II site of pSV2CAT-XB using Sau 3AI-Aat II adaptor (5'GATCACGT3') to obtain pF154CAT.

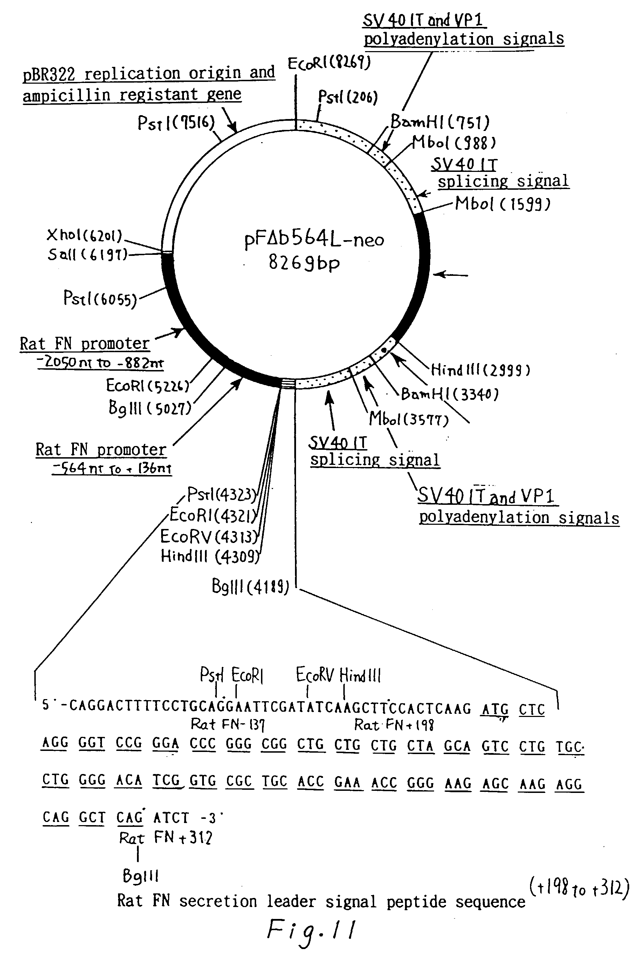

<u>pFΔa882CAT, pFΔa746CAT pFΔa564CAT, pFΔa414CAT and pFΔa123CAT(see Fig. 5; 11, 12, 13, 14 and 16)</u>

After digesting prFNsn2.3 with Eco RI, the resultant was blunt-ended with T4DNA polymerase and then Bgl II linker was attached. The resultant was digested with Bgl II and Xho I to obtain a DNA fragment of 1 kbp (containing -1908 nt to -1075 nt of FN enhancer). The DNA fragment was inserted between the Bgl II site and the Xho I site of pSV2CAT-BX to obtain pSV2CAT-FNa.

The Bgl II-Hind III fragments containing the FN promoter and enhancer were inserted from pF882CAT, pF746CAT, pF564CAT, pF414CAT and pF123CAT, and each of the DNA fragments were inserted between the Hind III site and Bgl II site of pSV2CAT-FNa to obtain pFΔa882CAT, pFΔa746CAT, pFΔa564CAT, pFΔa414CAT and pFΔa123CAT, respectively.

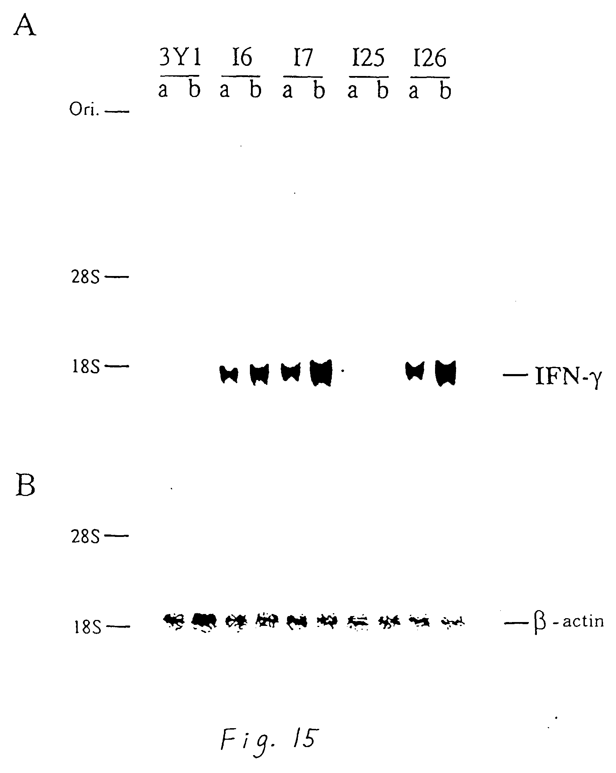

<u>pFΔa154CAT (see Fig. 5; 15)</u>

Plasmid prFNpp2.1 was digested with Aat II and Hind III to obtain a DNA fragment of 300 bp (containing -154 nt to +136 nt of the 5' flanking region of FN). The DNA fragment was inserted between the Hind III site and the Bgl II site of the pSV2CAT-FNa prepared in the preceding paragraph using Sau 3AI-Aat II adaptor (5'GATCACGT3') to obtain pFΔa154CAT.

<u>pFΔb746CAT and pFΔ564CAT (see Fig. 5; 17 and 18)</u>

Plasmid prFNsn2.3 was digested with Stu I and a Bgl II linker was attached. The resultant was digested with Bgl II and Xho I to obtain a DNA fragment of 1.1 kbp (containing -1908 nt to -882 nt of FN enhancer). The DNA fragment was inserted between the Bgl II site and the Xho I site of pSV2CAT-BX to obtain pSV2CAT-FNb.

The Bgl II-Hind III fragments containing the FN promoter and enhancer were isolated from pF746CAT and pF564CAT, respectively, and the obtained fragments were inserted between the Hind III site and the Bgl II site of pSV2CAT-FNb to obtain pFΔb746CAT and pFΔb564CAT, respectively.

<u>pFΔc564CAT (see Fig. 5; 19)</u>

After digesting prFNsn2.3 with Apa LI, the resultant was blunt-ended with T4DNA polymerase and a Bgl II linker was attached. The resultant was digested with Bgl II and Xho I to obtain a DNA fragment of 1.3 kbp (containing -908 nt to -742 nt of FN enhancer). The DNA fragment was inserted between the Bgl II site and Xho I site of pSV2CAT-BX to obtain pSV2CAT-FNc.

Bgl II-Hind III fragment containing the FN promoter and enhancer was isolated from pF564CAT and was inserted between the Hind III site and the Bgl II site of pSV2CAT-FNc to obtain pFΔc564CAT.

<u>pFgGGCAT, pFggGCAT, pFgGgCATA and pFgggCAT (see Fig. 5; 20, 21, 22 and 23)</u>

In order to strengthen the functions of the FN promoter and enhancer, substitutions of bases were introduced into three G-rich regions, that is, the regions located at -239 nt to - 229 nt, -105 nt to -95 nt and -54 nt to -44 nt, which consist of 10 G (guanine) or 9 G plus 1 C (cytosine) at the center of the 10 bases (these regions are hereinafter referred to as "G10 regions"), so as to prepare the pFCATs described in this section and the next section.

Plasmid pF564CAT was digested with Bgl II and Hind III to obtain a DNA fragment of 720 bp containing FN promoter. This DNA fragment was inserted between the Bam HI site and the Hind III site of M13mp19 phage vector to obtain M13F564 phage. <u>E. coli</u> CJ236 was infected with M13F564 phage to prepare a single-stranded phage DNA. Using this single-stranded phage DNA as a template and using synthetic DNA primers and site-specific mutation-introducing system "Mutan-K" commercially available from Takara Shuzo, the three GGGG sequences located at (a) -236 nt to -233 nt, (b) -98 nt to -95 nt and (c) -54 nt to -51 nt, respectively, were substituted by ATCC, ATCC and CTTA, respectively, by the method of Kunkel et al. By these substitutions, the G10 regions were destructed.

Among the base-substituted mutated phages, the phage in which (a) alone was substituted was designated M13F<u>gGG</u>, the phage in which (a) and (b) were substituted was designated M13<u>ggG</u>, the phage in which (a) and (c) were substituted was designated M13F<u>gGg</u>, and the phage in which (a), (b) and (c) were substituted was designated M13F<u>ggg</u>. Thus, the character "g" in the underscored portion means the substituted mutation and the character "G" in the underscored portion means the wild type sequence. Further, the first "g" indicates the position of (a), the second "g" indicates the position of (b) and the third "g" indicates the position of (c).

From M13FgGG, M13FggG, M13FgGg and M13Fggg, pFgGGCAT, pFggGCAT, pFgGgCAT and pFgggCAT were constructed, respectively, in the same manner as the construction of pF414CAT.

pFΔGCAT, pFΔGgCAT and pFΔggCAT (see Fig. 5; 24, 25 and 26)

From M13ggG, M13gGg and M13ggg prepared in the preceding section, pFΔggGCAT, pFΔGgCAT, and pFΔggCAT were constructed, respectively, in the same manner as in the construction of pF123CAT. The character "Δ" in the underscored portion means that the sequence (a) is deleted.

(2)-2 CAT Assay

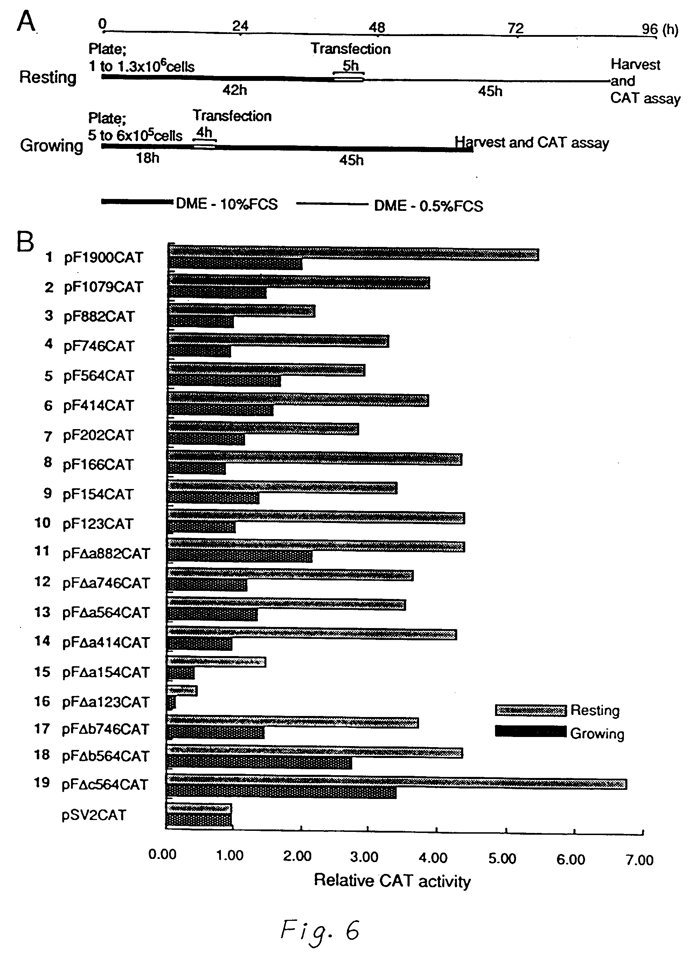

Expression in 3Y1 Cells (see Fig. 6)

Using the 26 pFCATs prepared in the preceding sections, CAT-expressing plasmid ptkCAT (Hayashi et al., Genes & Development, 1, 818) as a control and pSV2CAT, CAT assays were carried out on the growing 3Y1 cells and quiescent 3Y1 cells in low-serum medium containing 0.5 vol% of bovine serum (Fig. 6A). The transfection of the DNA was carried out under the following conditions: Growing 3Y1 Cells: About $5 \times 10^5$ cells were scattered on each petri dish with a diameter of 9 cm. On the next day, DNA was given to the cells in an amount of 20 μg per dish by calcium phosphate co-precipitation method (Chen et al., Molecular and Cellular Biology, 7(8), 2745) for 4.5 - 6 hours.
Quiescent 3Y1 Cells: About $1 \times 10^6$ cells were scattered on each petri dish with a diameter of 9 cm. Two days after, the transfection of the DNA was carried out in the same manner as for growing 3Y1 cells just mentioned above.

In both cases, the cells were harvested at 48 - 50 hours after the transfection. The cells were subjected to freeze-thaw cycle three times in 0.25 M Tris-HCl buffer (pH 7.8) to obtain cell extracts.

The CAT assay was carried out according to the method of Gorman et al. The results are shown in Fig. 6B. As shown in Fig. 6B, the following was found. In the following description, the pFCATs are designated in terms of the number shown in Fig. 5.

(a) From the entire results, it can be seen that FN promoter functioned more strongly in quiescent 3Y1 cells than in the growing 3Y1 cells.
(b) From the entire results, it can be seen that FN enhancer and promoter have higher expression activity than those of tk promoter and SV2 promoter.
(c) From the comparisons between 1 and 2, and between 3 - 6 and 11 - 14, it was found that a region which enhances the expression is contained in the sequence from -1908 nt to -1079 nt of the FN enhancer.
(d) From the comparisons between 1 and 18, and 2-4 and 5, a region which suppresses the expression is contained in the sequence from -882 nt to -564 nt of the FN enhancer.
(e) From the comparisons between 6, 14 and 9, 15 or 10, 16, it was found that a region which is necessary for the functioning of the expression-enhancing region contained in -1908 nt to -1079 nt described in (c) exists in the sequence between -414 nt and -154 nt.

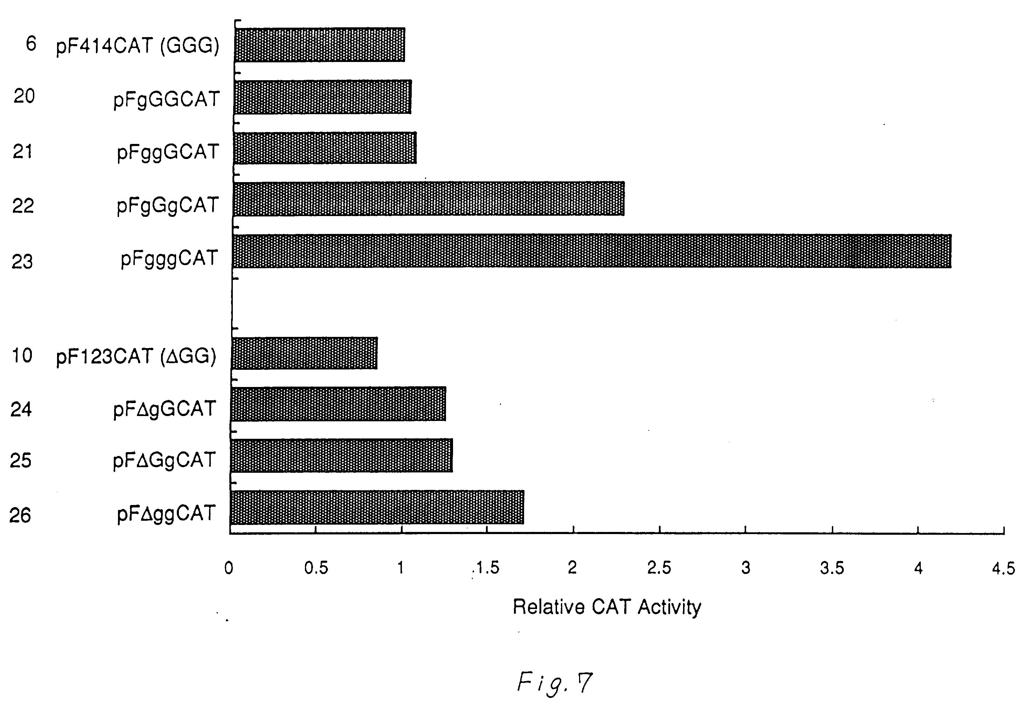

Expression in Transformed 3Y1 Cells (XhoC14 Cells) (Fig. 7)

In cell line XhoC14 prepared by transforming 3Y1 cells with adenovirus E1 gene, the expression of fibronectin is much lower than the normal 3Y1 cells. Using XhoC cells, CAT assays were carried out as in the preceding section. As a result, the expression of pFCATs of 1 - 19 were as low as about 1/10 of those in 3Y1 cells (The conditions of transfection and the method of CAT assay were the same as for the growing 3Y1 cells in the preceding section). On the other hand, pFCATs of 20 - 26 (see Fig. 5) to which base-substitution mutations were introduced in the G10 regions were transfected to XhoC cells and CAT assays were carried out. The expression levels were compared with pF414CAT (=pFGGGCAT) and pF123CAT (=pFΔGGCAT) to obtain the results shown in Fig. 7. From these, the following was found:

(f) The three G-rich regions (G10 regions) contained in the FN promoter and enhancer suppress the expression in the transformed cell line (XhoC).
(g) Therefore, by destroying the three G10 regions by, for example, introducing mutations such as substitution of bases, the FN enhancer and promoter regain the strong expression activity even in the transformed cell line.

(3) Construction of Vectors pF1900L, pFΔb564L, pF1900Lneo and pFΔb564neo for Transduction

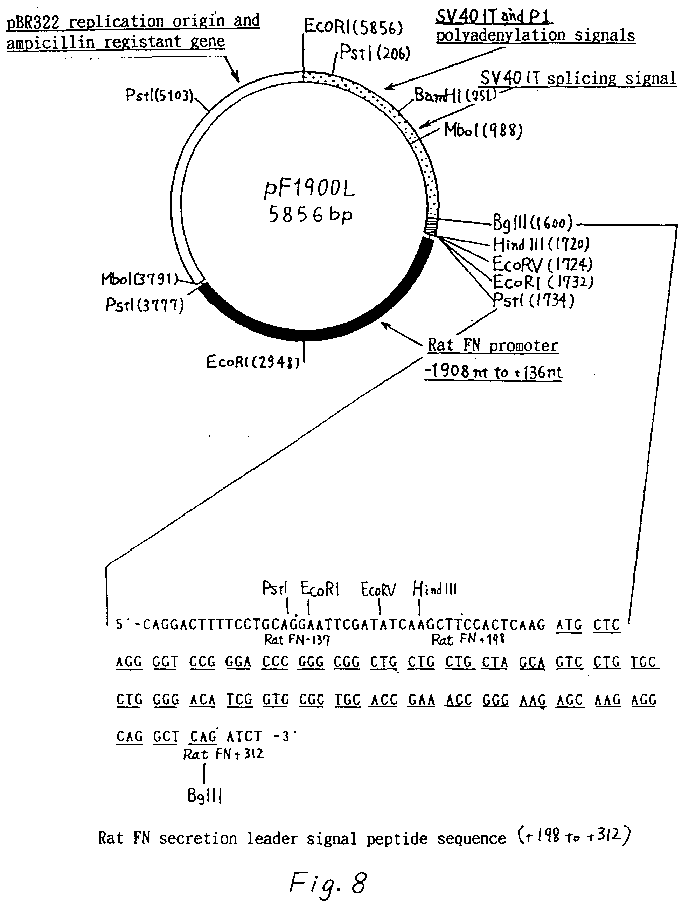

Based on the results described in the preceding sections, transducing vectors pF1900L, pFΔb564L, pF1900Lneo and pFΔb564Lneo were constructed (Fig. 8).

Plasmids pF1900L and pFΔb564L are the same as pF1900CAT and pFΔb564CAT, respectively, except that their CAT genes are replaced by +198 nt to +312 nt fragment (the region encoding FN secretion signal). The newly inserted FN secretion signal region has a Hind III site at the 5' end and a Bgl II site at 3' end, so that this region can be cut out by using these restriction enzymes. Further, these site can be used as cloning sites for an arbitrary structural gene. It should be noted, however, when a gene is cloned at the Bgl II site, the codon frame must be coincident with the amino acid code of the FN secretion signal.

Plasmids pF1900Lneo and pFΔb564Lneo were prepared by adding a Bam HI linker to the Nde I site of pSV2neo, digesting the resultant with Bam HI, and inserting the DNA fragment containing SV2 promoter and neomycin-resistant gene to the Bam HI site of pF1900L and pFΔb564L, respectively. After introducing these plasmids into cells, the stable transformants can be isolated as G418-resistant strains.

E. coli DH1 was transformed with pF1900L by the method described by Maniatis, T., Fritsch, E.F. and Sambrook, J. (1982), Molecular Cloning: A Laboratory Manual (Cold Spring Harbor, New York: Cold Spring Harbor Laboratory) to obtain a transformant E. coli AGI/pF1900L (FERM BP-3552).

Example 2

### (4) Construction of Transducing Vector pF1900M

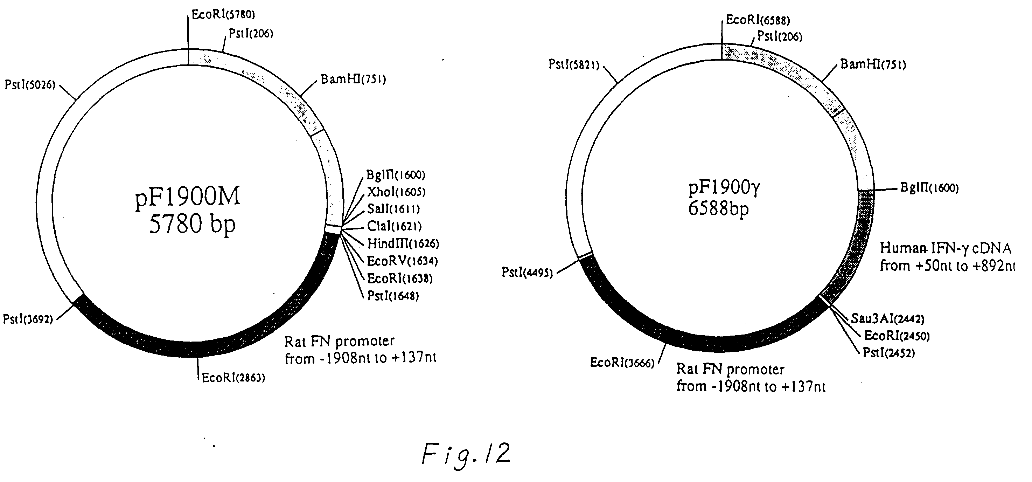

A vector pF1900M which does not preliminarily have the FN secretion signal was prepared separately from the transducing vectors constructed in the preceding sections (Fig. 12).

Firstly, the multicloning site adjacent to the 3' end of the FN enhancer and promoter of prFNpp2.1 was cut by Apa I. After inserting a Bgl II linker, the resultant was digested with Bam HI and Bgl II to isolate a DNA fragment containing the FN enhancer and promoter. Then pSV2β-globin was digested with Nde I and the resultant was treated with T4DNA polymerase, followed by insertion of the Bgl II linker. The resultant was digested with Bgl II to isolate a DNA fragment containing the splicing/polyadenylation signal of SV40, and a DNA fragment containing the replication origin of pBR322 and ampicillin-resistant gene. These isolated DNA fragments were ligated and a plasmid in which the splicing/polyadenylation signal of SV40 is arranged at the 3'-end of the FN enhancer and promoter was selected, which was designated pF1900M.

### (5) Production of Gene Product Using pF1900M

For the purpose of examining the ability to produce the gene product of the thus prepared expression vector pF1900M, human interferon gamma (hereinafter referred to as "HuIFN-γ") gene was introduced into the rat 3Y1 cells using pF1900M to establish a HuIFN-γ-expressing cell lines I series (I6, I7, I25 and I26). Using these cell lines, the level of the produced HuIFN-γ in the serum-free or low-serum medium, the expression level of mRNA, the state of existence of the introduced gene in the genome of the cell and the produced HuIFN-γ were examined.

From the analytical results, the following five points were confirmed. From these results, it was shown that it is useful to culture a mammalian cell transformant to which a structural gene is introduced by using the transducing vector pF1900M so as to produce the gene product.

(i) The amount of the gene product by the mammalian cell transformant to which a structural gene is introduced by using the transducing vector pF1900M, which transformant is simply cultured in a single layer is greater than the amount of the gene product obtained by culturing the conventional mammalian cell transformants.
(ii) The amount of the gene product by the mammalian cell transformant to which a structural gene is introduced by using the transducing vector pF1900M is increased by stopping the growth of the cells.
(iii) The amount of the gene product by the mammalian cell transformant to which a structural gene is introduced by using the transducing vector pF1900M depends on the activity of the FN enhancer and promoter for expressing the mRNA of the structural gene.
(iv) In the mammalian cell transformant to which a structural gene is introduced by using pF1900M, about 3 - 6 copies of the introduced gene are retained.
(v) The gene product produced by the mammalian cell transformant to which a structural gene is introduced by using pF1900M is similar to the naturally occurring gene products with respect to the modification by sugar chains and the like.

The concrete methods and results of the analysis will now be described.

### (5)-1 Insertion of HuIFN-γ cDNA to Downstream of pF1900M FN Enhancer and Promoter (Preparation of pF1900γ)

Plasmid pF1900M was digested with Eco RV and a Bgl II linker was inserted, followed by the digestion with Bgl II to obtain a vector fragment. Then HuIFN-γ cDNA presented by Dr. Shigekazu OSADA, Chief of Molecular Biology Department in Bioscience Research, Osaka University was cut at the Sau 3AI sites located at the 50th and 892th base from the mRNA transcription initiation site (Gray et al., Nature (London) 295, 503-508 (1982)), and the obtained DNA fragments were ligated to the pF1900M vector fragment. The plasmid in which the 5'-end of HuIFN-$\gamma$ cDNA is adjacent to the 3'-end of the FN promoter was selected to obtain a HuIFN-$\gamma$ expression plasmid pF1900$\gamma$.

(5)-2 Establishing HuIFN-$\gamma$-Producing Cell Lines I6, I7, I25 and I26

About $5 \times 10^5$ 3Y1 cells were scattered on a petri dish with a diameter of 8 cm and the cells were cultured overnight. To the 3Y1 cells, 20 $\mu$g of pF1900$\gamma$ prepared in the preceding section and 2 $\mu$g of a drug resistant gene-expression plasmid pSV2neo were administered for 4 hours by the calcium phosphate co-precipitation method (Chen, et al., Molecular and Cellular Biology, 7, 2745-2752 (1987)). After the administration, 3Y1 cells were cultured in Dulbecco's modified Eagle medium containing 10 vol% of fetal calf serum (FCS) (hereinafter referred to as "10%FCS-DMEM") for 48 hours, and then the cells were scattered on fresh 4 petri dishes. For about two weeks from the re-scattering, the cells were cultured on 10%FCS-DMEM containing 300 $\mu$g/ml of G418 (geneticin). The emerged colonies were isolated using a cloning cup, and were cultured in 10%FCS-DMEM containing 300 $\mu$g/ml of G418 (geneticin). After subculturing for about 4 weeks, the medium was changed to 10%FCS-DMEM which did not contain G418 (geneticin) and the cells were subcultured once to obtain I cell line.

The culture supernatant of each of the I cell lines obtained was collected and the HuIFN-$\gamma$ level therein was determined by the anti-virus assay against mengo virus. That is, in the wells of a 96-well culture plate, human FL cells were placed in the number of $5 \times 10^4$ cells/well and 100 $\mu$l of 5%FCS-DMEM and 45 $\mu$l of serially diluted standard HuIFN-$\gamma$ or the collected culture supernatant of I cell line were placed in each well, followed by culturing for 24 hours. To each well, $1 \times 10^5$ mengo virus was added and the cells were cultured for another 24 hours. Thereafter, the supernatant was discarded and the cells in the wells were stained with crystal violet. From the concentration of the HuIFN-$\gamma$ required for the FL cells to acquire anti-viral activity and the dilution of the culture supernatant of I cell line, which has the comparable activity, the HuIFN-$\gamma$ level in the supernatant was calculated.

Among the I cell lines, I6, I7, I25 and I26 cell lines which exhibited the largest production amount of the HuIFN-$\gamma$ secreted to the culture supernatant were selected and used for the studies below described.

(5)-3 Influences by Culturing Method on HuIFN-$\gamma$ Production by I Cell Lines

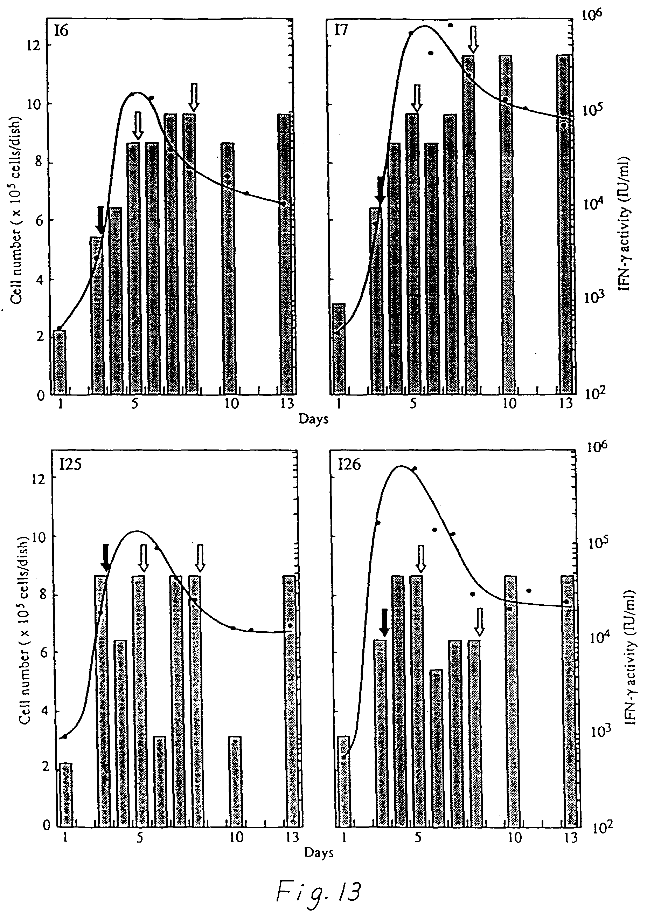

On a petri dish with a diameter of 5 cm, $2 \times 10^5$ cells of I6, I7, I25 or I26 were scattered ($1 \times 10^4$ cells/cm$^2$), and the cells were cultured in 4 ml of 10%FCS-DMEM for 3 days. The medium was then changed to 4 ml of the fresh medium with the same composition and the culturing was continued for another two days. The cells attached to the petri dish were washed twice with PBS-, and then cultured in 4 ml of DMEM containing no FCS (0%FCS-DMEM). The medium was again changed to 4 ml of 0%FCS-DMEM and the cells were cultured for additional 5 days. During this culture, the number of cells and the HuIFN-$\gamma$ level (anti-viral activity titer) in the culture supernatant were measured for each I cell line. The results are shown in Fig. 13. The number of cells are indicated by the black points and solid line and the HuIFN-$\gamma$ level in the culture supernatant is indicated by the gray bar graph. The black arrow indicates the change of the medium to 10%FCS-DMEM, and the white arrow indicates the change of the medium to 0%FCS-DMEM. With all cell lines, the cells grew to confluency ($1 - 1.2 \times 10^6$ cells/dish ($5 - 6 \times 10^4$ cells/cm$^2$)) in about 5 days from the initiation of the culture, and decreased to $8 \times 10^5$ cells/dish ($4 \times 10^4$ cells/cm$^2$) in 5 days from the change of the medium to 0%FCS-DMEM. Thereafter, the cells were not substantially decreased for 3 days. The HuIFN-$\gamma$ level in the culture supernatant raised to about $5 \times 10^4$ units/ml in I6 and I7 cell lines in one day after the cell density reached to confluency. In particular, I7 cell line exhibited the maximum level of $4 \times 10^5$ units/ml for 2 days after the decrease of the number of the cells. The amounts of HuIFN-$\gamma$ per one cell of I6 and I7 cell lines were 0.03 units/cell/day and 0.05 units/cell/day on average, respectively, between the third day and the fifth day during the cell growth stage, and 0.06 units/cell/day and 0.12 units/cell/day on average, respectively, between the fifth day and eighth day during the cells did not grow (These amounts were calculated from the amount of the accumulated HuIFN-$\gamma$ for 24 hours based on the hypothesis that HuIFN-$\gamma$ is not decomposed with time and accumulates in the culture supernatant, and from the cell number estimated for every 24 hours).

From these results, it was found that the production ability of HuIFN-$\gamma$ is doubled at least in I6 and I7 cell lines. These results are coincident with the results of the CAT assays showing that the activity of the FN promoter is increased to about 2 - 5 times in the quiescent 3Y1 cells than in the growing cells (Fig. 6B).

(B) Influence of FCS Concentration in Medium on HuIFN-$\gamma$ Production

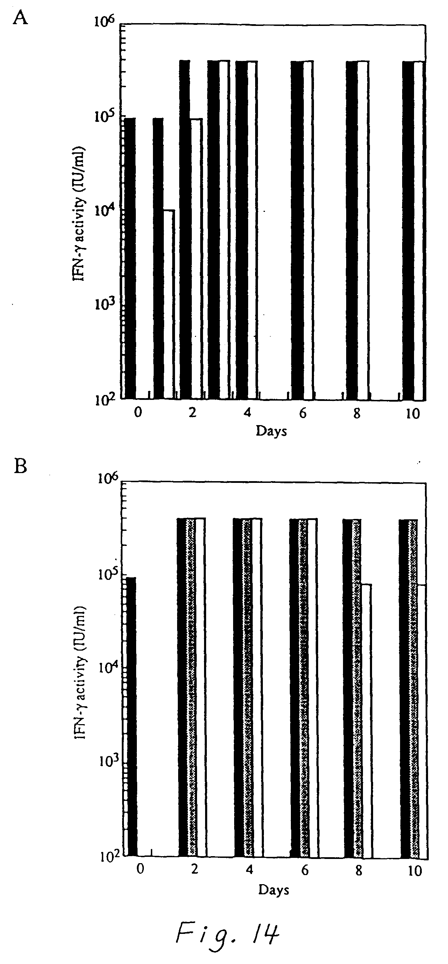

I7 cells were scattered on petri dishes with a diameter of 8 cm at a population density of $5 \times 10^5$ cells/dish ($1 \times 10^4$ cells/cm$^2$), and the cells were cultured for 3 days in 10 ml/dish of 10 vol% FCS-DMEM. The medium was then replaced with fresh medium with the same composition and the cells were cultured for another three days. Thus, the cells were cultured totally for 6 days, by which the cells reached to confluency ($6 \times 10^4$ cells/cm$^2$). On the 6th day from the beginning of the culture, sampling was initiated (Day0). That is, 100 μl of the culture supernatant was taken from each petri dish and the samples were stored at 4°C. The petri dishes were grouped into two groups. After washing the petri dishes twice with PBS-, 10 ml of 10%FCS-DMEM was added to each of the petri dishes of one group and 10 ml of 0%FCS-DMEM was added to each of the petri dishes of another group. The cells were cultured for 10 days without changing the media. On Day1, 2, 3, 4, 6, 8 and 10, 100 μl each of the culture supernatant was collected from each of the petri dishes, and the samples were stored at 4°C. The HuIFN-γ levels (antiviral activity titers) of the samples were measured, which are shown in Fig. 14A.

In the supernatant of the culture medium on Day0 (i.e., the culture medium in which the cells were cultured for 3 days in 10 ml of 10%FCS-DMEM), the HuIFN-γ level was $1 \times 10^5$ units/ml (black bar graph). In the group of cells which were further cultured in 10 ml of 10%FCS-DMEM, the HuIFN-γ level in the supernatant was $1 \times 10^5$ units/ml on Day1, and $5 \times 10^5$ units/ml on Day2 and thereafter. The reason why the HuIFN-γ level did not change after Day2 is assumed that the production and secretion of HuIFN-γ and the adsorption and decomposition of HuIFN-γ reached to an equilibrium.

On the other hand, the group to which 10 ml of 0%FCS-DMEM was given showed HuIFN-γ level of $1 \times 10^4$ units/ml on Day1, $1 \times 10^5$ units/ml on Day2, and $5 \times 10^5$ units/ml on Day3 and thereafter (white bar graph).

Microscopic observations revealed that in both groups, the population density was smaller on Day 10 than on Day 0, but the shrinkage of the cells and the collective peeling off of the cells were not observed.

These results indicate that (b) the expression of HuIFN-γ by the FN enhancer and promoter of pF1900M is not substantially influenced by FCS concentration contained in the medium for culturing the cells, and that (c) the expression of HuIFN-γ by the FN enhancer and promoter of pF1900M is promoted after the population density reaches to confluency and so the growth of the cells is stopped.

(C) Change in Concentration of Produced HuIFN-γ When Medium is Replaced Every Other Day

I7 cells were scattered on petri dishes with a diameter of 8 cm at a population density of $5 \times 10^5$ cells/dish ($1 \times 10^4$ cells/cm$^2$), and the cells were cultured for 3 days in 10 ml of 10%FCS-DMEM. The medium was then replaced with fresh medium with the same composition and the cells were cultured for another three days. Thus, the cells were cultured totally for 6 days, by which the cells reached to confluency ($6 \times 10^4$ cells/cm$^2$). On the 6th day from the beginning of the culture, sampling was initiated (Day0). That is, 100 μl of the culture supernatant was taken from each petri dish and the samples were stored at 4°C. The petri dishes were grouped into three groups. After washing the petri dishes twice with PBS-, 10 ml of 10%FCS-DMEM was added to each of the petri dishes of the first group, 10 ml of 0.5%FCS-DMEM was added to each of the petri dishes of the second group, and 10 ml of 0%FCS-DMEM was added to each of the petri dishes of the third group. The cells were cultured for 10 days changing the media to 10 ml each fresh medium having the same composition every other day. At each time of changing the medium, the supernatant of the old culture medium was taken and stored at 4°C. The HuIFN-γ levels (antiviral activity titers) in the samples were measured, which are shown in Fig. 14B.

In the supernatant of the culture medium on Day0 (i.e., the culture medium in which the cells were cultured for 3 days in 10 ml of 10%FCS-DMEM), the HuIFN-γ level was $5 \times 10^5$ units/ml (black bar graph). In the first group to which 10 ml of 10%FCS-DMEM was added (black bar graph) and in the second group to which 10 ml of 0.5%FCS-DMEM was added (gray bar graph), the HuIFN-γ level was $5 \times 10^5$ units/ml at every measurement up to Day10. In the third group to which 10 ml of 0%FCS-DMEM was added, although the HuIFN-γ level was $5 \times 10^5$ units/ml up to Day6, it was decreased to $1 \times 10^5$ units/ml on Day8 and Day10 (white bar graph).

Microscopic observations revealed that in the first and second groups to which 10 vol% and 0.5%FCS-DMEM media were given, respectively, the population densities on Day10 were only slightly smaller than those on Day0. However, in the third group to which 0%FCS-DMEM was given, it was observed that some cells died and shrunk and collectively peeled off on and after Day6.

These results indicate that (d) the concentration of the produced HuIFN-γ can be maintained at the $5 \times 10^5$ units/ml which is the maximum level even if the medium is replaced every other day, that (e) the production level of HuIFN-γ does not depend on the FCS concentration and that (f) when the DMEM is replaced every other day, about 0.5 vol% of FCS should be added in order to sustain the cells.

The results of this section ((5)-3) indicate that the amount of the HuIFN-γ produced by I7 cells by the simple monolayer culture in a low-serum medium is more than the amount of the HuIFN obtained by culturing mammalian cell transformants (E. Sano et al., in Biotechnology of Mammalian Cells (M. Umeda, et al., ed.) pp.149-162, Japan Scientific Societies Press, Tokyo (1987), R. Fukunaga, et al., Proceedings of the National Academy of Science USA, 81, 5086-5090 (1984)).

(5)-4 State of Expression of HuIFN-γ mRNA in I Cell Lines

The state of expression of the mRNA of HuIFN-γ in I cell lines was analyzed by Northern blot hybridization method. Firstly, from cell lines 3Y1, I6, I7, I25 and I26, total RNAs were extracted by acid guanidium phenol chloroform (AGPC) method (P. Chomczynski and N. Sacchi, Analytical Biochemistry, 162, 156-159 (1987)). The details of the procedure will now be described.

3Y1, I6, I7, I25 and I26 cells were cultured on plastic petri dishes with a diameter of 80 mm to a population density of $3 \times 10^6$ cells/dish (confluency), the number of the petri dishes for each cell line being 30. The petri dishes for each cell line were grouped into two groups (groups A and B, 15 dishes each). After washing the cells twice with PBS-, the petri dishes of group A were subjected to the preparation of mRNA. To the petri dishes of group B, 10 ml each of 0%FCS-DMEM was added and the cells were cultured for another 48 hours to completely stop the cell growth. Thereafter, the petri dishes of group B were subjected to the preparation of the RNA. The preparation of RNA was carried out by the following method (AGPC method):

The cells washed twice with cold PBS- (in the state of being attached to the petri dishes) were peeled off with Rubber Policeman and the cells were collected group by group (15 dishes per group) in a Falcon#2070 tube. The samples were then centrifuged at 1200 x g for 5 minutes and PBS- was removed by aspiration. To the resulting precipitation of the cells, 1 ml of denaturing solution (4M guanidiumthiocyanate, 25 mM sodium citrate, pH 7, 0.1 M β-mercaptoethanol and 0.5 wt% N-lauroylsarcosine (Sarkosyl) was added. The mixture was homogenized by repeating injection and aspiration through a 18 gauge needle using a 1 ml disposable syringe (commercially available from Terumo Corporation, Tokyo, Japan) until the mixture lost viscosity. To the homogenate, 0.1 ml of 2 M sodium acetate, pH 4, 1 ml of water-saturated phenol and 0.2 ml of chloroform were added and the resultant was vigorously stirred by a vortex mixer. The resulting mixture was incubated in ice for 15 minutes and was poured into two 1.5 ml centrifugation tube (commercially available from Eppendorf). The mixture was centrifuged at 12,000 x g at 4 ºC for 15 minutes. The separated aqueous layer was transferred to a new centrifugation tube and twice volume of isopropanol was added thereto. The resultant was incubated at -20°C for 1 hour to precipitate RNAs. The mixture was then centrifuged at 12,000 x g at 4 ºC for 15 minutes and the supernatant was discarded. The precipitate was dissolved in 300 μl of the denaturing solution and the mixture was then centrifuged for two minutes to remove the insoluble materials. The supernatant was transferred to a new centrifugation tube and equal volume of isopropanol was added. The resultant was incubated at -20°C overnight to precipitate RNAs, and then centrifuged at 12,000 x g, at 4°C for 15 minutes. The supernatant was discarded and the precipitate was rinsed with 75 vol% ethanol. The precipitate was dried in vacuum for two minutes and then dissolved in 25 μl of TE to obtain an RNA solution. The RNA concentration was determined by measuring the absorbance at 260 nm (O.D. 260) of a solution prepared by diluting 3 μl of the RNA solution with TE to 600 μ (i.e., 200-fold dilution), and calculated from the measured value (λ) according to the following equation:

$$\text{(RNA Concentration } \mu g/\mu l) = \lambda \times 40 \times 200 \div 1000 \text{ (wherein 1 O.D. 260 unit = 40 } \mu g/ml)$$

The extracted RNA was analyzed by Northern blot hybridization. The details of the procedure are as follows:

To 20 μg each of RNA extracted from 3Y1, I6, I7, I25 or I26 cell line, water was added to a volume of 4.5 μl. To the resulting mixture, 2 μl of 10 x MOPS electrophoresis buffer (a mixture of 200 mM sodium 3-(N-morpholino)-propanesulfonate, pH 7, 80 mM of sodium acetate and 10 mM of EDTA, pH 8.0), 3.5 μl of 12.3 M formaldehyde and 10 μl of formamide were added and the resultant was well stirred. The resultant was heated at 65°C for 15 minutes so as to denature the RNAs. To the denatured RNA solution, 5 μl of 6 x sample buffer (6 x formaldehyde loading buffer (a mixture of 1 mM EDTA, pH 8.0, 0.25 wt% of bromphenol blue (BPB), 0.25 wt% of xylenecyanol and 50 vol% glycerol) was added and the resultant was subjected to electrophoresis in agarose/formalin/gel (a mixture of 1.2 wt% agarose, 2.2 M formaldehyde, 20 mM MOPS, pH 7, 8 mM sodium acetate and 1 mM EDTA, pH 8.0) in 1 x MOPS electrophoresis buffer at an electric field density of 4V/cm for 4 hours (the distance of the bromphenol blue after the electrophoresis from the original position ($M_{BPB}$) = 11 cm). The separated RNAs were transcribed to a nylon membrane (Hybond N+, commercially available from Amersham) using an aspirative transcription apparatus (VacuGene, commercially available from Pharmacia-LKB) at an aspiration pressure (VP) of 40 mm $H_2O$ to prepare Nothern blot.

The Northern blot was incubated in a hybridization reaction solution (a mixture of 50 vol% formamide, 5 x SSC (SSC means 150 mM NaCl/15 mM sodium citrate), 5 x Denhalt's solution (0.02 wt% Ficoll 400/0.02 wt% polyvinylpyrrolidone/0.02 wt% bovine serum albumin (BSA)), 0.5 wt% SDS, 0.2 mg/ml heat-denatured herring sperm DNA) at 42°C for 4 hours. Then HuIFN-γ cDNA probe ($1 \times 10^9$ cpm/μg) or rat β-actin cDNA probe ($1 \times 10^9$ cpm/μg), which were labelled with $^{32}$P by the random primer method, was added to $5 \times 10^5$ cpm/ml. The resultant was then incubated at 42°C for additional 18 hours. The excess hybridization reaction solution attached to the Northern blot was removed by washing the blot twice in 2 x SSC at room temperature for 10 minutes, twice with 2 x SSC/0.1 wt% SDS at 42°C for 20 minutes, and once with 0.2 x SSC/0.1 wt% SDS at 42°C for 20 minutes. The washed Nothern blot was wrapped with Saranwrap (commercially available from Asahi Chemical Industry Co., Ltd.) and the amounts of the RNA molecules and their length with which the probes specifically hybridized were analyzed by using Bioimage Analyzer BAS2000 System (commercially available from Fuji Photo Film).

The bands of the mRNA of HuIFN-γ (IFN-γ) and of the rat β-actin (β-actin) detected are shown in Fig. 15A and 15B, respectively. On the upper portion of the blot, the cell line and the group are shown for each lane. On the left side of the blot, the positions of 28S and 18S ribosome RNAs contained in the RNA sample are indicated and the name of probes are indicated on the right side of the blot. The fact that the differences in the densities of the bands of rat β-actin are small among all of the cell lines and groups (Fig. 15B) indicates that the amounts of the RNA samples placed in all lanes are almost uniform. Thus, the differences in the density of the bands of mRNA of HuIFN-γ (Fig. 15A) reflect the differences in the expression levels of the HuIFN-γ gene.

As shown in Fig. 15A, mRNA of HuIFN-γ was detected as a single band in all of the cell lines except for the parent 3Y1 cell line. The positions of the bands are in the vicinity of the low molecular side of the 18S ribosome RNA, which are substantially coincident with the position of the band of the mRNA of IFN-γ of human lymphocyte reported by Gray (P.W. Gray et al., Nature (London), 295, 503-508 (1982)). As for I6, I7 and I26 cell lines, the expression levels of HuIFN-γ mRNA were about the same when the population density reached to confluency ($3 \times 10^6$ cells/dish, group A). Further, in these cell lines, the expression levels of the HuIFN-γ when the cell growth was completely stopped by culturing the cells in 0%FCS-DMEM for additional 48 hours (group B) were 1.5, 5 and 3 times those of Group A. The expression level of HuIFN-γ mRNA of I25 was low in both groups A and B, which were 0.2 and 0.3 times that of group A of I7 cell line.

The results of this section were coincident with the results of section (5)-3 (i.e., the HuIFN-γ productions by I cell lines are increased after the growth of the cells stopped). Therefore, it is apparent that the amount of the HuIFN-γ produced by I cell lines depends on the expression activity of FN enhancer and promoter.

(5)-5 State of Existence of pF1900M DNA Introduced in I Cell Lines

The states of existence of the pF1900M DNAs introduced into the I cell lines were analyzed by Southern blot hybridization method. Firstly, large genome DNAs were separated from 3Y1, I6, I7, I25 and I26 cell lines. The details of this procedure are as follows:

3Y1, I6, I7, I25 and I26 cells were cultured on plastic petri dishes with a diameter of 80 mm to a population density of $3 \times 10^6$ cells/dish (confluency), the number of the petri dishes for each cell line being 10. The cells were then washed twice with 10 ml/dish of PBS-, and lysed with 1 ml/dish of a mixture of 10 mM Tris-HCl, pH 7.4, 10 mM EDTA, 150 mM NaCl, 0.4 wt% sodium dodecyl sulfate (SDS) containing 1 mg/ml of bovine pancreas proteinase K (commercially available from Sigma) (Such a solution is hereinafter referred to as "proteinase K solution"). The cell lysate was collected to 50 ml plastic centrifugation tubes with screw caps and incubated at 60°C for 15 minutes and then at 37°C overnight while slowly inverting the dish. On the next day, 3 ml of a proteinase K solution containing 10 mg/ml of proteinase K was added to each centrifugation tube and the resultant was incubated at 60°C for 15 minutes and then at 37°C for two nights while slowly inverting the dish so as to decompose the proteinous components in the cells. After the incubation, the cell lysate was extracted 6 times with a mixture of salt saturated phenol (ss-phenol): chloroform (=1:1) and twice with chloroform, and the water layer was separated. In the extraction operations, the liquid layers were gently mixed so that the large molecular DNAs are not cut. To the aqueous layer, 1/10 volume of 3 M sodium acetate pH7.4 and 2.5 volume of ethanol were added so as to precipitate the genome DNAs. The DNAs were scooped with a tip of Pipetteman and rinsed with 5 ml of 80% ethanol. After drying the DNAs in the air for 5 minutes, the DNAs were redissolved in 5 ml of a mixture of 10 mM Tris-HCl, pH 7.4 and 0.5 mM EDTA solution (TE) at 37°C. It took one night to redissolve the DNAs. Thereafter, 100 μl of 10 mg/ml of ribonuclease A (RNase A) was added and the resultant was incubated at 37°C for one hour to decompose RNAs in the solution. The resultant was gently extracted with 5 ml of a mixture of salt saturated phenol: chloroform (=1:1). The aqueous layer was separated and re-extracted with chloroform. After separating the aqueous layer, 0.5 ml of 3 M sodium acetate, pH 7.4 and 13 ml of ethanol were added thereto, and the mixture was stirred. The precipitated DNAs were rinsed with 80% ethanol and dried in the air for 5 minutes, followed by dissolving in 2 ml of TE to obtain a genome DNA solution. The DNA concentration was determined by measuring O.D. 260 of a solution prepared by diluting 50 μl of the DNA solution with TE to 1 ml and by calculating the DNA concentration from the measured value (Y) according to the following equation:

$$(\text{DNA Concentration } \mu g/\mu l) = Y \times 50 \times 20 \div 1000 \text{ (wherein 1 O.D. 260 unit} = 50 \ \mu g/ml)$$

The thus obtained large molecular genome DNAs were digested with restriction enzyme Bgl II and the state of existence of the pF1900M DNA introduced into the genome was analyzed by Southern blot hybridization method as follows:

Forty micrograms each of the genome DNA solution of each cell line was completely digested with 80 units of Bgl II (commercially available from New England Biolabs) and the resultant was subjected to 0.5 wt% agarose gel electrophoresis in TBE buffer under an electric field density of 7.5 V/cm for 4 hours (the distance of the bromphenol blue after the electrophoresis from the original position ($M_{BPB}$) = 15 cm). The separated DNAs were transcribed to a nylon membrane (Hybond N+, commercially available from Amersham) using an aspirative transcription apparatus (Vacu-Gene, commercially available from Pharmacia-LKB) at an aspiration pressure (VP) of 40 mm $H_2O$ to prepare Southern blot.

The Southern blot was incubated in a hybridization reaction solution (a mixture of 50 vol% formamide, 5 x SSC, 5 x Denhalt's solution, 0.5 wt% SDS, 0.2 mg/ml heat-denatured herring sperm DNA) at 42°C for 4 hours. Then HuIFN-$\gamma$ cDNA probe (1 x $10^9$ cpm/$\mu$g) labelled with $^{32}$P by the random primer method was added to 5 x $10^5$ cpm/ml. The resultant was then incubated at 42°C for additional 18 hours. The excess hybridization reaction solution attached to the Southern blot was removed by washing the blot twice in 2 x SSC at room temperature for 10 minutes, twice with 2 x SSC/0.1 wt% SDS at 42°C for 20 minutes, and once with 0.2 x SSC/0.1 wt% SDS at 42°C for 20 minutes. The washed Southern blot was wrapped with Saranwrap (commercially available from Asahi Chemical Industry Co., Ltd.) and the amounts of the RNA molecules and their length with which the probes specifically hybridized were analyzed by using Bioimage Analyzer BAS2000 System (commercially available from Fuji Photo Film).

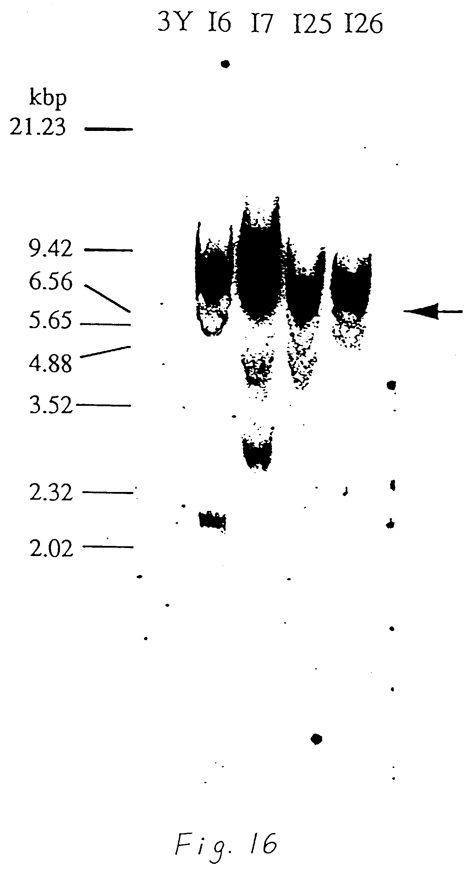

The states of the existence of pF1900M DNA on the Southern blot are shown in Fig. 16. The cell lines from which the genome DNAs were extracted are indicated on the upper portion of the blot for each lane, and the positions of the DNA length markers electrophoresed in the same gel are indicated on the left portion of the blot. The arrow on the right portion of the blot indicates the position of the linear pF1900M DNA (the unit chain length of pF1900M DNA) prepared by cutting pF1900M at one site by Bgl II.

For all of the cell lines except for the parent cell line 3Y1, the bands showing the specific hybridization with the probes were observed. The number of the bands of I6, I7, I25 and I26 cell lines are 3, 6, 8 and 7, respectively. In these I cell lines, the band of the largest copy number indicate the unit chain length of pF1900M DNA, which is 6 copies for I7 and 3 copies for other cell lines. These results suggest that the pF1900M DNA introduced in the parent 3Y1 cell line is inserted in the genome DNA of the cell maintaining substantially the full length.

(5)-6 HuIFN-$\gamma$ Protein Produced by I Cell Lines

Naturally occurring HuIFN-$\gamma$ has a molecular weight of about 22 - 25 kd (Y.K. Yip, et al., Proceedings of the National Academy of Science USA, 79, 1820-1824 (1982)), and the peptide portion thereof encoded by mRNA has a molecular weight of about 17.1 kd (P.W. Gray, et al., Nature (London), 295, 503-508 (1982)). The remaining 5 - 8 kd is occupied by sugar chains attached to the peptide by N-glycosyl bond via aspargine residue. In order to compare the HuIFN-$\gamma$ produced by I cell lines with the naturally occurring HuIFN-$\gamma$ (reported data), the HuIFN-$\gamma$ was separated from the culture supernatant by immunoprecipitation method, and analyzed by SDS-polyacrylamide gel electrophoresis. The details of the procedure will now be described.

The immunoprecipitation method employed was a modification of the method of Harlow et al (E. Harlow, D. Lane (ed.), Antibodies; a laboratory manual, 421-470, Cold Spring Harbor Laboratory Press (1988)). As the HuIFN-$\gamma$ cell lines, I6 and I7 were employed, and the parent 3Y1 cell line was employed as a control.

In petri dishes with a diameter of 50 mm, 3Y1, I6 and I7 cells were cultured to 1.2 x $10^6$ cells/dish (population density of 6 x $10^4$ cells/cm$^2$, confluency), and then cultured overnight in 0%FCS-DMEM. The medium was discarded and the cells attached to the petri dishes were washed twice with PBS. Five milliliters of 0% FCS-EMEM (Eagle's minimum essential medium) containing no methionine and 59 $\mu$l (18.6 MBq) of 7.5 $\mu$M[$^{35}$S]-methionine (41.8 TBq/mmol, New England Nuclear, Code No. NEG-072) were added to each dish and the cells were cultured at 37°C for 17 hours under 5% $CO_2$. To 1 ml of each culture supernatant, 4 $\mu$l of normal rabbit serum was added and the mixture was incubated at 4°C for 6 hours. Then 4 $\mu$l of SAC (Staphylococcus aureus Cowan I) pellet was added to each mixture and the bacterial cells were well suspended. The mixture was incubated in ice for 40 minutes and then centrifuged at 12,000 x g at 4°C for 5 minutes to remove the precipitate. To each supernatant, 5 $\mu$l of anti-HuIFN rabbit polyclonal antibody (commercially available from Hayashibara Biochemical Laboratories, Inc., Code No. PIF-3) (7 $\mu$g; 850 neutralization units) was added and the resulting mixture was incubated at 4°C for 3 hours. To each resulting mixture, 20 $\mu$l of protein A beads (Affi-Gel protein A, commercially available from BioRad, Code No. 153-6153) was added and the resultant was gently rocked at 4°C for 40 minutes, followed by centrifugation at 10,000 x g at 4°C for 15 seconds. After discarding the supernatant, the pellet was washed four times with 1 ml of cold PBS- and the beads were suspended in 40 $\mu$l of a mixture of 0.2 M Tris-HCl, pH 7.8, 1 wt% SDS and 100 mM of $\beta$-mercaptoethanol. The suspension was incubated at 85°C for 10 minutes to dissociate the antigen-antibody complex from the protein A beads. The resultant was then centrifuged at 10,000 x g at room temperature for 15 seconds and the supernatant was collected to obtain an immunoprecipitation sample of HuIFN-$\gamma$.

An aliquote of the immunoprecipitation sample was treated with N-glycanase (peptide-N$^4$-(N-acetyl-$\beta$-glucosaminyl)asparaginemidase, an enzyme which specifically cuts N-glycosyl bond, commercially available from Genzyme Co.,

Code No. 1472-00) to remove the sugar chains (F. Maley, et al., Analytical Biochemistry, 180, 195-204 (1989)).

Ten microliters of the immunoprecipitation sample was heated in boiled water for 5 minutes and then cooled in ice. To the resultant, 5 µl of 7.5 vol% Nonidet P-40, 1.2 µl of 0.25 U/µl N-glycanase and 13.8 µl of water were added and the resulting mixture was incubated overnight at 37°C. On the next day, 30 µl of 2 x Laemmli's sample buffer (a mixture containing 2 wt% SDS, 10 vol% glycerol, 100 mM dithiothreitol (DTT), 60 mM Tris-HCl, pH 6.8 and 0.001 wt% of bromphenol blue (BPB)) was added and the resultant was boiled for 10 minutes. The resultant was subjected to 16% SDS-polyacrylamide gel electrophoresis (concentration gel: 4.83 wt% monoacrylamide/0.17 wt% bis-acrylamide/125 mM Tris-HCl, pH 6.8/0.1 wt% SDS; separation gel: 15.4 wt% monoacrylamide/0.53 wt% of bis-acrylamide/ 375 mM Tris-HCl, pH 8.8/0.1 wt% SDS; electrophoresis buffer: 25 mM Tris/250 mM glycine/0.1 wt% SDS, pH 8.3; electrophoresis conditions: 100 V, 6 hours). On the other hand, to 10 µl of the immunoprecipitate sample which was not treated with N-glycanase, 10 µl of 2 x Laemmli's sample buffer was added and the resultant was boiled for 10 minutes. The resultant was used as a control.

After the electrophoresis, the gels were immersed in fluorescent amplifying solution (EN$^3$HANCE, Code No. NEF-981, commercially available from New England Nuclear) for one hour and dried at 70°C after washing with water. The gels were exposed to an X-ray film at -70°C for 2 hours.

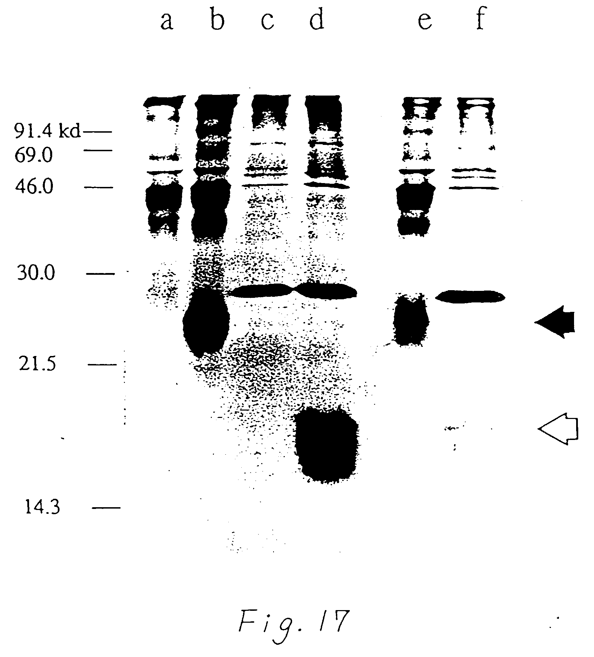

The results are shown in Fig. 17. The immunoprecipitation sample applied to each gel was as follows: Lane a; 3Y1 culture supernatant, lane b; I7 culture supernatant, lane c; 3Y1 culture supernatant (treated with N-glycanase), lane d; I7 culture supernatant (treated with N-glycanase), lane e; I6 culture supernatant (treated with N-glycanase), lane f; I6 culture supernatant (treated with N-glycanase). On the left portion of the drawing, the molecular weights of the marker proteins separated in the gel are indicated. On the right portion of the drawing, the assumed position of the naturally occurring HuIFN-γ (M.W. about 22 kd; black arrow) and the assumed position of the naturally occurring HuIFN-γ from which the sugar chains are removed (M.W. about 17.1 kd; white arrow). Since the proteins of the bands located at the arrows do not exist in the immunoprecipitation sample of the culture supernatant of the parent 3Y1 cells, they are apparently the products of HuIFN-γ gene introduced in the I cells as pF1900γ.

These results indicate that the HuIFN-γ produced by I6 and I7 cells is a molecule which comprises 17.1 kd of the polypeptide to which sugar chains are attached via N-glycosyl bond, which shows overall molecular weight of 22 - 25 kd. Therefore, it was proved that the HuIFN-γ produced by at least I6 and I7 cells is very similar to naturally occurring HuIFN-γ.

Although the invention was described by way of a preferred embodiment thereof, it is apparent for those skilled in the art that various modifications can be made within the spirit and scope of the present invention.

A recombinant vector by which a desired structural gene may be expressed efficiently in mammalian cells cultured in serum-free or low-serum medium is disclosed. The recombinant vector of the present invention comprises a cloned DNA having an enhancer activity for fibronectin gene.

## Claims

1. A cloned DNA sequence derived from rat genomic DNA having enhancer activity for the rat fibronectin gene, said DNA sequence comprising the nucleotide sequence shown in Fig. 3.

2. A recombinant vector comprising the DNA according to claim 1 operably linked to a promoter and a structural gene.

3. A process for producing a gene product, comprising the step of culturing mammalian cells transformed with the recombinant vector according to claim 2, and collecting the gene product produced by the expression of said structural gene.

4. The process according to claim 3, wherein the amount of said gene product is increased by culturing said mammalian cells in a serum-free or low-serum medium.

## Patentansprüche

1. Klonierte DNA-Sequenz, abgeleitet von genomischer DNA der Ratte, mit Verstärkeraktivität für das Fibronectin-Gen der Ratte, wobei die DNA-Sequenz die Nukleotidsequenz umfaßt, die in Fig. 3 dargestellt ist.

2. Rekombinierter Vektor, umfassend die DNA nach Anspruch 1, funktionsbereit mit einem Aktivator und einem Strukturgen verknüpft.

3. Verfahren zur Herstellung eines Genproduktes, umfassend den Schritt, daß Säugetierzellen kultiviert werden, die mit dem rekombinierten Vektor nach Anspruch 2 transformiert wurden, und Sammeln des Genproduktes, das durch Ausprägung des Strukturgens produziert wurde.

4. Verfahren nach Anspruch 3, worin die Menge des Genproduktes vergrößert wird, indem die Säugetierzellen in einem serumfreien oder serumarmen Medium kultiviert werden.

**Revendications**

1. Séquence d'ADN clonée dérivée d'un ADN génomique de rat, présentant une action d'activateur pour le gène de fibronectine de rat, ladite séquence d'ADN comprenant la séquence de nucléotides représentée sur la figure 3.

2. Vecteur recombinant comprenant l'ADN suivant la revendication 1 lié de manière fonctionnelle à un promoteur et un gène structural.

3. Procédé pour la préparation d'un produit de gène, comprenant l'étape consistant à cultiver des cellules de mammifère transformées avec le vecteur recombinant suivant la revendication 2, et à recueillir le produit de gène formé par l'expression du gène structural.

4. Procédé suivant la revendication 3, dans lequel la quantité du produit de gène est accrue en cultivant les cellules de mammifère dans un milieu sans sérum ou un milieu à basse teneur en sérum.

Fig. 1

EP 0 546 333 B1

Rat FN 5' flanking region
PstI fragment
(FN nucleotide position)

Pstl 705 (-1908)
Sau3AI 718 (-1895)

Rsal 1154 (-1459)

AatII 1334 (-1279)

EcoRI 1534 (-1079)
Rsal 1576 (-1037)

StuI 1731 (-882)

ApaLI 1867 (-746)
SmaI 1878 (-735)
Rsal 1957 (-656)
SmaI 2010 (-603)
SmaI 2049 (-564)

DraI 2199 (-414)

Sau3AI 2411 (-202)
Rsal 2447 (-166)
AatII 2459 (-154)
EagI 2490 (-123)

Pstl 2749 (+136)

Rat FN 5' flanking region
Sall/Nhel fragment
(FN nucleotide position)

Xbal/Nhel 677 (+243)
Smal 692 (+228)

Pstl 784 (+136)

EagI 1043 (-123)
AatII 1074 (-154)
Rsal 1086 (-166)
Sau3AI 1122 (-202)

DraI 1334 (-414)

SmaI 1484 (-564)
SmaI 1523 (-603)
Rsal 1576 (-656)
SmaI 1855 (-735)
ApaLI 1866 (-746)

StuI 1802 (-882)

Rsal 1957 (-1037)
EcoRI 1999 (-1079)

AatII 2199 (-1279)

Rsal 2379 (-1459)

Sau3AI 2815 (-1895)
Pstl 2828 (-1908)

Sall 2970 (-2050)

f1 (+) origin
LacZ
BssHII 619
Xbal 677
BamHI 689
MCS
Pstl
HindIII 2763
SalI 2778
BssHII 2836
Ampicillin'
prFNpp2.1
5008 bp
LacI
ColE1 ori

f1 (+) origin
LacZ
BssHII 619
Xbal/Nhel 677
MCS
Sall 2970
BssHII 3028
Ampicillin'
prFNsn2.3
5200 bp
LacI
ColE1 ori

Fig. 2

```
                                                              -1908
                                                              CTGCCAGGT
-1900  CAACGGATCT  GCCATATTTG  TGGAAAGCCG  TCCCCTCAGG  TTTATCTAGC  AAGCATCCTG  ATTTCCAGGT  ATCCTTCTCT  CCCCTGGGAGA  GAGCACTTCC
-1800  CAGAACTTCA  CTGTTTTACA  AGGAGCCCTT  TTATATCTGA  AGAGAAAAAC  CGTTTTGTCA  AGGGATGATG  AGAAATTCCA  AAAAACTAAG   TCAGACGGGA
-1700  TTCTAAATTT  GGGGGAATGT  ATGGCATTCG  TTCAACCCCG  AGTGTTGGTC  ACAAAGATTC  TCTACTTATT  TTACACCGCT  TGTAAGAAAC   TGGTATTCGT
-1600  TGTGGGTTTT  CTTGTTTTTA  GTTCTTTACA  TCAATAAAAA  TAAATGTGTT  TTTGCCTCT   GAGGATTTTA  TAAAGCATAC  AAATTCAAAT
-1500  TATCCTTACA  GCGGAACTTT  TGGCGAAAAC  TACAAATAAC  GGTAGCTTTA  ATATTTTGCT  GGTAGCTTTA  AAAGCAAATG  AGACCACGTG
-1400  CCTACCGAAG  GAGAAAGCCA  AGTTGGGAGT  TCAGACCTGC  TGCTTTGAAA  ACGTGTTAGA  TCTCATTTTG  AAGCACAGCA  ACTGGGCTGT  GTGTGTCTGA
-1300  AAGGTAAAAA  AAAAAGAAAA  GAAAAAAAAG  AAAAAAAAAA  AAAAAGACCA  ACGTGTTAGA  TCAGGGGACG  TCTCCTATCC  CCGCTCCAAT
-1200  CTCAGTAGTG  TAGAAAGTCG  GAGAATTTCC  GGGACTTTT   CAGTGGAACT  TTATACATTC  TTGGAGCAGA  TAACAATTGA  AATCTAAAAA  ATCTGGCGGC
                       Puel et al.
-1100  GAGGGAGGGG  GTGGATAGTG  GAATTCCGGG  GAAGGCTGGA  GGAGGCAAG   CCCACCCCAG  TGTACCCATC  CTCTCTTCA   GAGGCCACCT
-1000  AGTCTCTGCA  TTTGAGTGCA  AATGGCCCAG  AACGGGCACC  TGAACGTGGC  GTGGACCTAA  AGCTACCCGC  TCTCACCCGC  TCCCCCCTCT
 -900  CAAGCTGTTC  ACCACAGGCC  TTCTAAAAGA  TTCTAAAGA   CCAATCCCAG  GAAAGGAAAA  CTTTGTTTCA  TGACTTAGAA  AGGAAACTTT
 -800  ATCTTTGCAG  AGAGACTTGG  TCTTGTGAGT  TTGCCCGAA   TCTTCAGGGC  GCCGTGCACA  ACCCCGGGCT  GTAGAAGCTC  AGGGAGGGGG  TCACCTTTGA
 -700  CACGCGCTCA  CCTCTCCGGG  GCTCTCCCTC  GAAACCAAGC  AGGTACTGCA  TGGAGAAACC  GGGGTCTAAG  CCTACCTAAC  GGCGCCCGGG
 -600  ACCCAGGAAA  AGCACCAGGA  AGAGCCGCCC  TGCCCGGGGG  CTGCAAAAGT  GAAGTCTGGA  TTCCTACAGG  GACAAGGTAG  ACGACTTTT
 -500  CCCTTCCCAC  AAAATACACC  CCGGTGAGCA  CAGACTTTTC  TCAGAGGTGA  CGCAATGTTC  TCAAACACCA  CCACGGTCAC  AAAAAAAAA
 -400  AAAGGAAAGA  GACGAGGGGG  TAAACCTGTC  CCCTACCCCC  AGGCTTCAGT  ACGACCGCAA  CCTCCCTCCC  GTCTACTTCA  CTTTACAGCC
 -300  GTTTCCCATC  CCTACCCCCA  ATCCTCCTCC  CAAAAGTTTG  ACGACCGCAA  AGGAAACCAA  TTTCCTCCGA  AGTCCCAGAC  CTGGGGGAGA
 -200  TCAGCATCTC  TTTTGTTCGG  GGGCGAACCA  CCGTACCCCG  TGACGTCACC  CGGACTCGGC  [CCAATCGGCG]  CGCGCTGCGG  CAGGAG[GGGG]
 -100  [CGGGGGAGTC]  GGACGGGACC  CTCCTCCCCG  GCGCGGCAGGG  CCTCGTGGGG  [GGCGGG]AAG  GGACTGTCCC  ATATAAGCCT  GGCTCAGCCG
  +1   CTCGCACCCG  CTGCACTGCA  CAGGGGAAGA  AAAGGAGCCC  AGGGTGTGAG  GCCACAACTT  CGGGCCAGCG  CCGTGTCCT   CCTTCCATCT
                                          +137
 +101  TCTTTACAGGC  GTCCCCACCT  CAGGACTTTT  CTGCAG
```

*Fig. 3*

19

Fig. 4

Fig. 5

Fig. 6

*Fig. 7*

pBR322 replication origin and ampicillin registant gene

EcoRI(5856)

PstI(206)

SV40 IT and P1 polyadenylation signals

SV40 IT splicing signal

BamHI(951)

MboI(988)

PstI(5103)

pF1900L
5856 bp

BgIII(1600)
Hind III(1720)
EcoRV(1724)
EcoRI(1732)
PstI(1734)

MboI(3791)
PstI(3777)

EcoRI(2948)

Rat FN promoter
-1908nt to +136nt

PstI    EcoRI    EcoRV    HindIII

5'-CAGGACTTTTCCTGCAGGAATTCGATATCAAGCTTCCACTCAAG ATG CTC

Rat FN-137                    Rat FN+198

AGG GGT CCG GGA CCC GGG CGG CTG CTG CTG CTA GCA GTC CTG TGC

CTG GGG ACA TCG GTG CGC TGC ACC GAA ACC GGG AAG AGC AAG AGG

CAG GCT CAG ATCT -3'
Rat FN+312

BgIII

Rat FN secretion leader signal peptide sequence (+198 to +312)

Fig. 8

pBR322 replication origin and
ampicillin registant gene

SV 40 IT and VP1
polyadenylation signals

EcoRI (8444)

PstI (206)

BamHI (751)

MboI (988)

SV40 IT splicing signal

MboI (1599)

PstI (7691)

MboI (6379)

PstI (6365)

*pF 1900L-neo*
*8445 bp*

EcoRI (5536)

Rat FN promoter
-1908 nt to +136 nt

PstI (4322)
EcoRI (4320)
EcoRV (4312)
HindIII (4308)

BglII (4188)

SV40 IT
splicing signal

HindIII (2999)

BamHI (3340)

MboI (3577)

SV40 IT and VP1
polyadenylation signals

PstI  EcoRI      EcoRV  HindIII

5'-CAGGACTTTTCCTGCAGGAATTCGATATCAAGCTTCCACTCAAG ATG CTC

Rat FN -137                  Rat FN +198

AGG GGT CCG GGA CCC GGG CGG CTG CTG CTG CTA GCA GTC CTG TGC

CTG GGG ACA TCG GTG CGC TGC ACC GAA ACC GGG AAG AGC AAG AGG

CAG GCT CAG ATCT -3'

Rat FN +312

BglII

Rat FN secretion leader signal peptide sequence (+198 to +312)

*Fig. 9*

<u>pBR322 replication origin and</u>
<u>ampicillin registant gene</u>

EcoRI(5680)
PstI(206)

<u>SV 40 IT</u>
<u>polyadenylation signals</u>

PstI(4327)

BamHI(751)

MboI(988)

<u>SV 40 IT splicing signal</u>

pFΔb564L
5680 bp

BglII (1600)
Hind III (1720)
EcoRV (1724)
EcoRI (1732)
PstI (1734)

<u>Rat FN promoter</u>
<u>-564 nt to +136 nt</u>

XhoI (3612)
SalI (3608)
PstI (3466)
<u>Rat FN promoter</u>
<u>-2050 nt to -882 nt</u>

BglII (2438)

EcoRI (2697)

```
                    PstI  EcoRI   EcoRV  HindIII
                     |  .  |       |     |
5'-CAGGACTTTTCCTGCAGGAATTCGATATCAAGCTTCCACTCAAG ATG CTC
          Rat FN-137                Rat FN+198

AGG GGT CCG GGA CCC GGG CGG CTG CTG CTG CTA GCA GTC CTG TGC

CTG GGG ACA TCG GTG CGC TGC ACC GAA ACC GGG AAG AGC AAG AGG

CAG GCT CAG ATCT -3'
        Rat FN +312
         |
        BglII
```

Rat FN secretion leader signal peptide sequence (+198 to +312)

*Fig. 10*

Rat FN secretion leader signal peptide sequence (+198 to +312)

Fig. 11

Fig.12

Fig. 13

A

B

Fig. 14

A

3Y1    I6    I7    I25    I26
‾‾‾‾   ‾‾‾   ‾‾‾   ‾‾‾‾   ‾‾‾‾
a  b   a  b  a  b  a  b   a  b

Ori. —

28S —

18S —　　　　　　　　　　　　　　　　　　　— IFN-γ

B

28S —

18S —　　　　　　　　　　　　　　　　　　　— β - actin

Fig. 15

Fig. 16

Fig. 17